# EUROPEAN PATENT SPECIFICATION

(11) **EP 1 914 316 B1**
(45) Date of publication and mention of the grant of the patent: **16.12.2009**
(21) Application number: 07018460.1
(22) Date of filing: 31.07.2002
(51) Int. Cl.: C12Q 1/66, C12Q 1/02, A61K 49/00, C12N 15/03

(54) **LIVP strain of vaccinia virus for diagnosis and therapy of tumours**
LIVP Stamm von Vaccinia-Virus zur Diagnose und Behandlung von Tumoren
Souche LIVP de vaccinia virus pour le diagnostic et la thérapie des tumeurs

(30) Priority: 31.07.2001 EP 01118417; 30.10.2001 EP 01125911
(43) Date of publication of application: 23.04.2008
(62) Divisional of application: 02794632.6
(73) Proprietor: Genelux Corporation, San Diego, California 92109 (US)
(72) Inventor: Szalay, Aladar A., Highland, CA 92346 (US); Yu, Yong A., San Diego, CA 92130 (US); Shabahang, Shahrokh, Redlands, CA 92373 (US); Timirysova, Tatyana, San Diego, CA 92109 (US)
(74) Representative: Blance, Stephen John

(56) References cited:
- WO-A-01/05229
- WO-A-01/25399
- RODRIGUEZ J F ET AL: "EXPRESSION OF THE FIREFLY LUCIFERASE GENE IN VACCINIA VIRUS A HIGHLY SENSITIVE GENE MARKER TO FOLLOW VIRUS DISSEMINATION IN TISSUES OF INFECTED ANIMALS" PROCEEDINGS OF THE NATIONAL ACADEMY OF SCIENCES OF THE UNITED STATES, vol. 85, no. 5, 1988, pages 1667-1671, XP002194182 1988 ISSN: 0027-8424
- WEISSLEDER RALPH ET AL: "In vivo magnetic resonance imaging of transgene expression." NATURE MEDICINE, vol. 6, no. 3, March 2000 (2000-03), pages 351-354, XP002194183 ISSN: 1078-8956

## Description

The present invention relates to the use of an LIVP strain of vaccinia virus in the preparation of compositions suitable for the detection or treatment of a tumor, tumor tissue, cancer or metastasis in a subject, wherein the virus encodes a detectable protein or a protein that induces a detectable signal e.g. a luminescent or fluorescent protein, and, in a particular embodiment, furthermore (a) DNA sequence(s) encoding (a) protein(s) suitable for tumor therapy and/or elimination of metastatic tumors, e.g. a cytotoxic or cytostatic protein.

Presence of bacteria in tumors was reported approximately fifty years ago. Several publications substantiated the earlier clinical findings that unexpectedly large numbers of bacteria were discovered: in excised tumors from human patients. Investigators argue that chronic infections may predispose cells to malignant growth. Chronic infections of various strains of *Chlamydia* have been associated with lung and cervical cancer as well as malignant lymphoma. Another well described association between the presence of a specific bacterial species and cancer development is *Helicobacter pylori* in patients with gastric ulcers. Elevated levels *of H. pylori-associated* antibodies have been found in patients with duodenal ulcer and gastric adenocarcinoma. These observations demonstrate a concomitant presence of bacteria at tumor sites; however, it was not yet clear whether the microorganisms were the cause of tumor formation or whether the tumorous tissues were more susceptible to bacterial colonization. Intravenously injected strict anaerobic bacteria, *Clostridium pasteurianum,* into mice replicated selectively in the tumor suggesting a hypoxic microenvironment in the necrotic center. Intravenous injection of attenuated *Salmonella typhimurium* mutants resulted in elevated bacterial titers in the tumor tissues in comparison to the other organs of mice upon histologic and bacteriologic analyses.

Similarly, the presence of virus particles was reported in excised human breast tumors as early as 1965. More recently, based on polymerase chain reaction (PCR) data, the human papillomavirus has been claimed to be associated with anogenital tumors and esophageal cancers, breast cancers, and most commonly, cervical cancers. In addition, the presence of hepatitis C virus in human hepatocellular carcinoma, Epstein-Barr virus in squamous cell carcinoma in Kirnura's disease, mouse mammary tumor virus-like paiticles (MMTV) in human breast cancer, SV40 virus in macaque astrocytoma, and herpesvirus in turtle fibropapilloma has been reported. Surprisingly, the concentration of virus particles in the tumors shows variations among patients. The presence of human papillomavirus in squamous cell carcinomas of the esophagus ranges from 0 to 72% (10- 15). In contrast to tumor tissues, no virus particles have been found in tumor-free areas of the esophageal epithelium of the same patient suggesting that the virus particles are located only in the tumor tissues.

WO 0 125 399 A describes the use of EBV for the detection and treatment of B-lymphocytes.

However, so far it could not be shown definitely whether the above discussed microorganisms are responsible for the development of disorders like tumors (except for papillomaviruses) or whether, e.g., tumors can attract and/or protect viruses or bacteria. Accordingly, there was no basis for the use of such microorganisms for the diagnosis or therapy of tumors. Conventional tumor diagnostic methods, such as MRI (Magnetic Resonance Imaging) lack sensitivity and specificity and therapeutic methods, e.g. surgery, are invasive and not very sensitive.

Therefore, it is the object of the present invention to provide a means for the efficient and reliable diagnosis as well as the therapy of tumors which overcomes the disadvantages of the diagnostic and therapeutic approaches presently used.

According to the present invention this is achieved by the subject matters defined in the claims. When *Vaccinia* virus (LIVP strain) carrying the light emitting fusion gene construct *rVV-rvc-gfp* was injected intravenously into nude mice, the virus particles were found to be cleared from all internal organs within 4 days, as determined by extinction of light emission. In contrast, when the fate of the injected *Vaccinia* virus was similarly followed in nude mice bearing tumors grown from subcutaneously implanted C6 rat glioma cells, virus particles were found to be retained over time in the tumor tissues, resulting in lasting light emission. The presence and amplification of the virus-encoded fusion proteins in the same tumor were monitored in live animals by observing GFP fluorescence under a stereomicroscope and by collecting luciferase-catalyzed light emission under a low-light video-imaging camera. Tumor-specific light emission was detected 4 days after viral injection in nude mice carrying subcutaneous C6 glioma implants ranging in size from 25 to 2500 mm³. The signal became more intense after the 4th postinjection day and lasted for 30 to 45 days, indicating continued viral replication. Tumor accumulation of rVV-*ruc-gfp* virus particles was also seen in nude mice carrying subcutaneous tumors developed from implanted PC-3 human prostate cells, and in mice with orthotopically implanted MCF-7 human breast tumors. Further, intracranial C6 rat glioma cell implants in immunocompetent rats and MB-49 mouse bladder tumor cell implants in C57 mice were also targeted by the *Vaccinia* virus. Cross sections of a C6 glioma revealed that light emission was clustered in "patches" at the periphery of the tumor where the fast-dividing cells reside. In contrast, cross sections of breast tumors revealed that fluorescent "islands" were distributed throughout the tumors. In addition to primary breast tumors, small metastatic tumors were also detected externally in the contralateral breast region, as well as in nodules on the exposed lung surface, suggesting metastasis to the contralateral breast and lung. In summary, light-emitting LIVP strain of *Vaccinia* virus can be used to detect and treat primary and metastatic tumors.

Similar results were obtained with light-emitting bacteria (*Salmonella, Vibrio, Listeria, E. coli*) which were injected intravenously into mice and which could be visualized in whole animals under a low light imager immediately. No light emission was detected thirty-six hours after bacterial injection in both athymic (nu/nu) mice and immunocompetent C57 mice as a result of clearing by the immune system. In the cutaneous wound of an intravenously injected animal, the bacterial light emission increases and remains detectable up to six days post-injection. In nude mice baring tumors developed from implanted C6 glioma cells, light emission was abolished from the animal entirely thirty-six hours after delivery of bacteria, similar to mice without tumors. However, forty-eight hours post-injection, unexpectedly, a strong, rapidly increasing light emission originating only from the tumor regions was observed. This observation indicates a continuous bacterial replication in the tumor tissue. The extent of light emission is dependent on the bacterial strain used. The homing-in process together with the sustained light emission was also demonstrated in nude mice carrying prostate, bladder, and breast tumors. In addition to primary tumors, metastatic tumors could also be visualized as exemplified in the breast tumor model. Tumor-specific light emission was also observed in immunocompetent C57 mice with bladder tumors as well as in Lewis rats with brain glioma implants. Once in the tumor, the light-emitting bacteria were not observed to be released into the circulation and to re-colonize subsequently implanted tumors in the same animal. Further, mammalian cells expressing the Ruc-gfp fusion protein, upon injection into the bloodstream, were also found to home into and propagate in glioma tumors.

These findings open the way for (a) designing multifunctional viral vectors useful for the detection of tumors based on signals like light emission and/or for suppression of tumor development and/or angiogenesis signaled by, e.g., light extinction in combination with therapeutic gene constructs for the treatment of cancer. These systems have the following advantages: (a) They target the tumor specifically without affecting normal tissue; (b) the expression and secretion of the therapeutic gene constructs are, preferably, under the control of an inducible promoter, enabling secretion to be switched on or off; and (c) the location of the delivery system inside the tumor can be verified by direct visualization before activating gene expression and protein delivery.

Accordingly, the present invention relates to the use of a diagnostic and/or pharmaceutical composition comprising an LIVP strain of *Vaccinia* virus containing a DNA sequence encoding a detectable protein or a protein capable of inducing a detectable signal as defined in the claims.

In a preferred embodiment, the LIVP strain of *Vaccinia* virus of said diagnostic and/or pharmaceutical composition furthermore contains (a) DNA sequence(s) encoding (a) protein(s) suitable for tumor therapy and/or elimination of metastatic tumors, such as a cytotoxic protein, a cytostatic protein, a protein inhibiting angiogenesis or a protein stimulating apoptosis. Such proteins are well-known to the person skilled in the art and further examples of suitable proteins are given below.

The LIVP strain of *Vaccinia* virus is useful for the compositions used in the present invention, provided that it replicates in the organism, is not pathogenic for the organism, e.g. attenuated, and is recognized by the immune system of the organism, etc. Examples of useful microorganisms are bacteria and viruses. The term "bacteria" as used herein refers to bacteria which are *per se* not tumor-targeted (i.e. they can not differentiate between a cancerous cell or tissue and the non-cancerous counterpart cell or tissue) since the results of the experiments leading to the present invention show that bacteria etc. accumulate in the tumor due to the fact that in this environment they are not exposed to attack by the immune system of the host. A list of candidate bacteria which might be useful for the purposes of the present disclosure and which might not be tumor-targeted are given in Table 1, below. The person skilled in the art can easily identify such bacteria which are not tumor-targeted by commonly available methods, e.g. the methods described in section 6.1 of WO 96/40238. Preferably, said bacteria are intercellular bacteria such as *E*. *coli, E. faecalis, Vibrio cholerae, Vibrio fischeri, Vibrio harveyi, Lactobacillus spp., Pseudomonas spp.* In the method of the present invention, viruses are preferred which are not tumor-targeted.

In a particularly preferred embodiment, the diagnostic and/or pharmaceutical composition comprises an LIVP strain of *Vaccinia* virus containing a DNA sequence encoding a luminescent and/or fluorescent protein.

As used herein, the term "DNA sequence encoding a luminescent and/or fluorescent protein" also comprises a DNA sequence encoding a luminescent and fluorescent protein as fusion protein.

In an alternative preferred embodiment of the diagnostic and/or pharmaceutical composition, the protein encoded by the LIVP strain of *Vaccinia* virus is a cell receptor capable of binding a ligand which can be a diagnostic or therapeutic ligand. The ligand can be a protein (including large or small peptides, antibodies, etc.), a synthetic compound (such as a synthetic steroid analog), etc. Therefore, the *in vivo* location of labeled ligands in live animals and human patients can be visualized, e.g, in real time by SPECT or PET. Almost any known protein ligand-receptor pair for tumor labeling is useful in the method of the present invention. Preferably, to increase specificity, mutant protein ligands (or analogs if it is a chemical compound) or mutant ligand receptors can be genetically or chemically engineered so that they will not bind to any endogenous molecules. In addition to increasing specificity, these mutants/analogs will also limit adverse effect to the normal host physiology.

In a more preferred embodiment of the diagnostic and/or pharmaceutical composition used in the present invention, the ligand is a radionuclide-labelled ligand. Said ligand is, e.g., useful for tumor visualization by single-photon emission computed tomography (SPECT) or positron-emission tomography (PET) resulting from the binding of radionuclide-labelled ligand to its receptors expressed specifically on the surface of tumor cells following intravenous delivery of, e.g., engineered LIVP strain of *Vaccinia* virus carrying the receptor protein gene constructs. Radionuclides may be used for conventional tumor scintigraphy, PET and possibly internal radiotherapv of tumors. Examples of radionuclides useful in the present invention are (a) β⁺-emitters such as ¹¹C, ¹³N, ¹⁵O or ⁶⁴Cu or (b) γ-emitters such as ¹²³I. Other radionuclides that can, e.g., be used as tracers for PET include ⁵⁵Co, ⁶⁷Ga, ⁶⁸Ga, ⁶⁰Cu(II), ⁶⁷Cu(II), ⁵⁷Ni, ⁵⁵CO, ⁵²Fe, ¹⁸F, etc.

SPECT and PET are sensitive techniques that may be used for tumor imaging according to the present invention. Both SPECT and PET are capable of detecting trace amounts of β⁺ and γ emission from radionuclides. PET is even more sensitive than SPECT. In experiments using small laboratory animals, tumor imaging is performed using a microPET instrument, which is commercially available through, e.g., Concorde Microsystems (Knoxville, TN).

Examples of useful radionuclide-labeled agents are ⁶⁴Cu-labeled engineered antibody fragment (Wu et al., PNAS USA 97 (2002), 8495-8500), ⁶⁴Cu-labeled somatostatin (Lewis et al., J.Med.Chem. 42 (1999), 1341-1347, ⁶⁴Cu-pyruvaldehyde- bis(N4-methylthiosemicarbazone) (⁶⁴Cu-PTSM) (Adonai et al., PNAS USA 99 (2002), 3030-3035, ⁵²Fe-citrate (Leenders et al., J.Neural Transm. Suppl. 43 (1994), 123-132, ⁵²Fe/^{52m}Mn-citrate (Calonder et al., J.Neurochem. 73 (1999), 2047-2055) and ⁵²Fe-labeled iron(III) hydroxide-sucrose complex (Beshara et al., Br.J.Haematol. 104 (1999), 288-295, 296-302).

In order to apply the radionuclide-labeled ligand in tumor detection, the genes encoding the receptor proteins that the ligands may bind are delivered by an intravenously injected LIVP strain of *Vaccinia* virus, according to the present invention. Since it could be shown in the examples, below, that an intravenously injected LIVP strain of *Vaccinia* virus, replicates specifically in the tumors, expression of the receptor proteins in the tumors will mark the tumors for targeting by the radionuclide-labeled ligands.

For example, in the case of *Vaccinia* virus, to allow efficient tumor detection, the virus can be used to carry gene constructs encoding receptor proteins that can bind specifically to the ligands. Intravenously injection of recombinant *Vaccinia* virus allows the delivery of the receptor gene and surface expression of the receptor protein in the tumor tissues. Then, the radionuclide-labeled ligands are injected intravenously into the host. The specific binding between radionuclide-labelled ligands and their receptors expressed on the tumor cell surface allows the detection of tumors based on β⁺- or γ-emissions originating only from the tumors. Labelling the tumors with radionuclides will allow easy distinction between tumors and normal tissues. Therefore, handhold β⁺*-* or γ-detectors that are attached to a surgical blade holder can be designed. Based on the signal of β⁺- or γ-emission, the labelled tumors can be cleanly excised while the removal of normal tissues is kept at a minimum.

Particularly preferred is gallium-67 for the purposes of the present invention. Gallium-67 (⁶⁷Ga) can be used for diagnostic imaging using PET, SPECT, or scintigraphy. It is known for its ability to accumulate in inflammatory lesions and tumors, especially in lymphomas, but also in many other types of tumors, such as in pancreatic tumors, lung tumors etc. The mechanism of ⁶⁷Ga uptake has been proposed to be through both transferrin-dependent route and transferrin-independent route. In the transferrin-dependent route, it has been shown that over-expression of transferrin receptor significantly increases ⁶⁷Ga uptake by tumor cells. Furthermore, anti-transferrin receptor antibody significantly blocks ⁶⁷Ga by tumor cells. For small tumor imaging, very high concentrations of ⁶⁷Ga are required to overcome background signal. Thus, in this case, the use of recombinant *Vaccinia* virus carrying a transferrin receptor gene construct for over-expressing transferrin receptors specifically on the surface of tumor cells in live animals or human patients following intravenous injection of the viruses is preferred. ⁶⁷Ga will be also delivered intravenously. High level accumulation of ⁶⁷Ga in tumor cells with the help from the over-expressed transferrin receptors helps to significantly improve tumor detection abilities in live animals and human patients.

In an alternative preferred embodiment, the diagnostic and/or pharmaceutical composition used in the present invention comprises an LIVP strain of *Vaccinia* virus containing a DNA sequence encoding a protein capable of inducing a signal detectable by magnetic resonance imaging (MRI), e.g. metal binding proteins. Furthermore, the protein can bind contrast agents, chromophores, ligands or compounds required for visualization of tissues. Preferably, said protein is a cell receptor capable of binding a paramagnetic- or superparamagnetic metal-labelled ligand. Tumor visualization by MRI, resulting from the binding of paramagnetic- or superparamagnetic-metal-labelled ligands to their receptors expressed specifically on the surface of tumor cells following, e.g., intravenous delivery of engineeredviruses according to the present invention carrying the receptor protein gene constructs has several advantages. The high level of accumulation of these metals in the tumors will facilitate tumor detection. Virtually any paramagnetic or superparamagnetic metals can be used for this purpose. Preferably, due to the systemic toxicity of naked heavy metals, the paramagnetic or superparamagnetic are carefully selected to keep adverse effects at minimal. In most currently available contrast agents, the metal particles are either linked to chelates or are coated with polymers, which are much safer to use than naked particles. Therefore, the use of ligands tagged with chelated or polymer-coated metals is preferred.

Methods for generating contrast agents linked with proteins are well known to the person skilled in the art, e.g., the protein ligand (or other chemical compound ligand) is first chemically attached to the chelates (e.g. diethylene triamine pentaacetic acid (DTPA)). Then, the chelate-protein complex is labeled with metals. A similar labeling method has been used in generating gallium-labeled somatostatin analogs and used in producing indium-111-labeled low density lipoprotein using DTPA-bis(stearylamide). Details of protein modifications with chelates used in contrast agents have been previously described. For example, modifying proteins with the bifunctional chelate 2-(4-isothiocyanotobenzyl-6-methyl) diethylenetriamine-pentaacetic acid (MX-DTPA)) (Mirzadeh et al., Bioconjug. Chem. 1 (1990), 59-65) and modifying proteins with the cyclic anhydride of diethylenetriamine-pentaacetic acid (cDTPAA) (Duncan and Welch, J. Nucl. Med. 34 (1993), 1728-1738) have been described.

Alternatively, MRI can be carried out by the following approaches:

### (a) Modified gadolinium activation due to enzyme/protein delivery

This approach is based on the principles of Gene-Directed Enzyme Prodrug Therapy (GDEPT), in which case the enzymes/proteins activate a systemically delivered nontoxic prodrug into active toxic drug. Specific activation of the MRI contrast agent in weak relaxivity state into strong relaxivity state by the enzyme/protein in the tumors allows tumor detection. The enzyme/protein delivery to tumors is achieved through intravenously injected engineered bacteria, viruses, and mammalian cells, which carry the gene encoding β-galactosidase (or any other related enzymes). The β-galactosidase can be in either extracellular secreted form or expressed an the bacteria or mammalian cell surface. An example of an MRI agent that can be cleaved by β-galactosidase and used in MRI imaging for tumor detection is (4,7,10-tri(acetic acid)-1-(2-β-galactopyranosylethoxy)-1,4,7,10-tetraazacyclododecane) gadolinium (Egad) (Moats et al., 1997,). In this compound, a galactopyranose residue is positioned at the 9th coordination site of the Gd³⁺ ion. Due to this blockage, water protons are excluded from interacting with Gd³⁺ ion, and therefore diminished effect on T1 relaxation time. In the presence of β-galactosidase, the enzyme cleaves the galactopyranose from the chelate, freeing the coordination site, and allows irreversible transition of the contrast agent from a weak to a strong relaxivity state. Since the bacterium-, virus-, or mammalian cell-based enzyme expression occurs only in tumors, the enzyme-mediated relaxivity state transition will also occur only in the tumors. Therefore, this enzyme-mediated activation of MRI contrast agents can be used for tumor detection.

In addition to the above MRI contrast agent, similar new contrast agents may be developed in which other types of residues may be attached to the chelates. These residues can be removed by their corresponding enzymes (similar to the removal of galactopyranose by galactosidase) (e.g. α-mannosidases, α- and β-glucosidases, β-glucuronidases) to free the 9th coordination site of the Gd³⁺ ion and modify the T1 relaxation time for tumor detections. The genes encoding these enzymes may all be delivered by the engineered LIVP strain of *Vaccinia* viruses according to the present invention. Moreover, new contrast agents using metals other than gadolinium may be developed for enzyme-activated MRI tumor imaging.

This approach can be combined with therapy as shown in the following sections.

### (b) Engineering of delivery vector systems for activation of the contrast agents (e.g. modified gadolinium) first and subsequently, the activation of an injected prodrug by the same constitutively expressed enzyme (e.g. β-galactosidase) - One-Gene-Product-Based Detection and Therapy System (OGPBDTS)

For example, the gadolinium-based contrast agent can be activated by an enzyme (e.g. β-galactosidase), as described above, which can be used for tumor detection. Subsequently, the intravenously delivered prodrugs, such as CBI, TMI, PCI (described in US-patent 5,646,298), may be cleaved by the same β-galactosidase in the tumors, to yield active cytotoxic drugs against tumor cells for cancer treatment.

### (c) Engineering of delivery vector systems with the Two-Gene-Product-Based Detection and Therapeutic Systems (TGPBDTS)

Gene 1 is linked to a constitutive promoter and produces the enzymes/proteins for sensing the contrast agents (e.g. metal-binding proteins, modified gadolinium, or other agents) and gene 2 is linked to an exogenously activatable promoter, which is silent without the activator drug, and only turned on after the detection vector system is found positive by MRI. The activation of the vector-based promoter gene construct is achieved by injection or by oral administration of the activator drug. The repeated injection of the contrast agent allows the real-time monitoring of tumor size and location of metastases during the treatment procedure.

Preferably, the DNA sequences encoding the (diagnostic or therapeutic) proteins described above, e.g., a luminescent and/or fluorescent protein are present in a vector or an expression vector. A person skilled in the art is familiar with examples thereof. The DNA sequences can also be contained in a recombinant virus containing appropriate expression cassettes. The virus that may be used in the diagnostic or pharmaceutical composition of the present invention is an LIVP strain of *Vaccinia* virus. For expression in mammals, a suitable promoter is e.g. human cytomegalovirus "immediate early promoter" (pCMV). Furthermore, tissue and/or organ specific promoters are useful. Preferably, the DNA sequences encoding, e.g., a luminescent and/or fluorescent protein are operatively linked with a promoter allowing high expression. Such promoters, e.g. inducible promoters are well-known to the person skilled in the art.

For generating the above described DNA sequences and for constructing viruses which contain said DNA sequences, it is possible to use general methods known in the art. These methods include e.g. *in vitro* recombination techniques, synthetic methods and *in vivo* recombination methods as described in Sambrook et al., Molecular Cloning, A Laboratory Manual, 2nd edition (1989) Cold Spring Harbor Laboratory Press, Cold Spring Harbor, NY, for example. Methods of transfecting cells, of phenotypically selecting transfectants and of expressing the DNA sequences by using the above described vectors are known in the art.

The person skilled in the art knows DNA sequences encoding proteins, e.g., luminescent or fluorescent proteins that can be used in the diagnostic and/or pharmaceutical composition of the present invention. During the past decade, the identification and isolation of structural genes encoding light-emitting proteins from bacterial luciferase from *Vibrio harveyi* (Belas et al., Science 218 (1982), 791-793) and from *Vibrio fischerii* (Foran and Brown, Nucleic acids Res. 16 (1988), 177), firefly luciferase (de Wet et al., Mol. Cell. Biol. 7 (1987), 725-737), aequorin from *Aequorea victoria* (Prasher et al., Biochem. 26 (1987), 1326-1332), *Renilla* luciferase from *Renilla reniformis* (Lorenz et al., PNAS USA 88 (1991), 4438-4442) and green fluorescent protein from *Aequorea victoria* (Prasher et al., Gene 111 (1987), 229-233) have been described that allow the tracing of bacteria, viruses or mammalian cells based on light emission. Transformation and expression of these genes in bacteria allows detection of bacterial colonies with the aid of the low light imaging camera or individual bacteria under the fluorescent microscope (Engebrecht et al., Science 227 (1985), 1345-1347; Legocki et al., PNAS 83 (1986), 9080-9084; Chalfie et al., Science 263 (1994), 802-805).

Luciferase genes have been expressed in a variety of organisms. Promoter activation based on light emission, using *lux AB* fused to the nitrogenase promoter, was demonstrated in Rhizobia residing within the cytoplasm of cells of infected root nodules by low light imaging (Legocki et al., PNAS 83 (1986), 9080-9084; O'Kane et al., J. Plant Mol. Biol. 10 (1988), 387-399). Fusion of the *lux A* and *lux B* genes resulted in a fully functional luciferase protein (Escher et al., PNAS 86 (1989), 6528-6532). This fusion gene (*Fab2*) was introduced into *Bacillus subtilis* and *Bacillus megatherium* under the xylose promoter and then fed into insect larvae and was injected into the hemolymph of worms. Imaging of light emission was conducted using a low light video camera. The movement and localization of pathogenic bacteria in transgenic arabidopsis plants, which carry the pathogen-activated PAL promoter-bacterial luciferase fusion gene construct, was demonstrated by localizing *Pseudomonas* or *Ervinia spp.* infection under the low light imager as well as in tomato plant and stacks of potatoes (Giacomin and Szalay, Plant Sci. 116 (1996), 59-72).

All of the luciferases expressed in bacteria require exogenously added substrates such as decanal or coelenterazine for light emission. In contrast, while visualization of GFP fluorescence does not require a substrate, an excitation light source is needed. More recently, the gene cluster encoding the bacterial luciferase and the proteins for providing decanal within the cell, which includes *luxCDABE* was isolated from *Xenorhabdus luminescens* (Meighen and Szittner, J. Bacteriol. 174 (1992), 5371-5381) and *Photobacterium leiognathi* (Lee et al., Eur. J. Biochem. 201 (1991), 161-167) and transferred into bacteria resulting in continuous light emission independent of exogenously added substrate (Fernandez-Pinas and Wolk, Gene 150 (1994), 169-174). Bacteria containing the complete *lux* operon sequence, when injected intraperitoneally, intramuscularly, or intravenously, allowed the visualization and localization of bacteria in live mice indicating that the luciferase light emission can penetrate the tissues and can be detected externally (Contag et al., Mol. Microbiol. 18 (1995), 593-603).

A list of candidate bacterial strains that might be useful for the same purpose as the present invention (i.e. BMPT (bacterium-mediated protein therapy) and tumor imaging) and which might not be tumor-targeted are listed in Table 1. The person skilled in the art can easily identify such bacteria which are not tumor-targeted by commonly available methods, e.g. the methods described in section 6.1 of WO 96/40238.

For safety and direct applicability to humans, bacterial cell lines such as milk and cheese associated microorganisms are preferred which are naturally consumed by most individuals and have intrinsic anti-tumor activity when injected directly to solid tumors. Such bacteria also include bacteria that were naturally isolated from human tumors which developed co-existence (symbiosis) with a variety of types of tumors or with a specific type of tumor. Extracellular secretion of the therapeutic proteins by bacteria is mediated through either signal peptides or endogenous protein secretion pathways. To provide additional safety to the BMPT, bacterial inducible promoters, such as IPTG-induced lac promoter may be used to exogenously regulate therapeutic protein production by bacteria. IPTG-regulated expression of mammalian proteins in bacteria has been well documented. IPTG has also been shown to be functional in promoter activation in animals. In addition to *lac* promoter, other examples of promoters that allow regulation of gene expression in bacteria include the ara promoter (activated by arabinose) and PLtetO-1 promoter (activated by anhydrotetracycline (aTc)) (Lutz and Bujard, Nucleic Acids Res. 25 (1997), 1203-1210).

**Table 1. Examples of candidate bacterial strains which might be useful for the same purpose as the present invention:**

| **Bacterial strain** | **Brief description** | **Reference** |
|---|---|---|
| | | |
| Aerobes, gram positive | | |
| *Lactobacillus bulgaricus* | Yogurt bacteria; nonpathogenic (part of normal flora beneficial organism needed by human body); blastolysin fraction isolated from *L. bulgaricus* shows antitumor effect (Bogdanov et al. 1975; Bogdanov et al. 1977; Ketlinskii et al. 1987); may reduce the risk of developing colon tumors in humans (Wollowski et al. 2001); ATCC#11842. | Simonds et al. 1971, J Bacteriol 107:382-384; Bogdanov et al. 1975, FEBS Lett 57:259-261 ; Bogdanov et al. 1977, Biull Eksp Biol Med 84:709-712 ; Ketlinskii et al. 1987, Vopr Onkol 33:51-56; Wollowski et al. 2001, Am J Clin Nutr 73:451S-455S |
| *Lactobacillus casei* | Nonpathogenic (part of normal flora beneficial organism needed by human body); i.v. injected LC9018 strain of *L*. *casei* shows markedly inhibition of the growth of subcutaneously inoculated sarcoma-180 in mice (Kato et al. 1981); shows antitumor activity from i.p. injected *L. casei* (LC9018 strain) (Kato et al. 1988); ATCC# 393. | Kato et al. 1981, Gann 72:517-523; Kato et al. 1988, Cancer Immunol Immunother 26:215-221 |
| *Lactobacillus acidophilus* | Nonpathogenic (part of normal flora beneficial organism needed by human body); inhibitory effect on liver tumorigenesis (Mizutani and Mitsuoka 1980); reduces 1,2-dimethylhydrazine (DMH)-induced intestinal tumors in male Sprague-Dawley rats (McIntosh et al 1999); shows strong antiproliferative effect of milk fermented by *L. acidophilus* on the growth of a human breast cancer cell line (Biffi et al. 1997); | Mizutani and Mitsuoka 1980, Cancer Lett 11:89-95; Biffi et al. 1997, Nutr Cancer 28:93-99; McIntosh et al. 1999, Nutr Cancer 35:153-159 |
| *Lactobacillus brevis* | Nonpathogenic (part of normal flora beneficial organism needed by human body); | |
| *Lactobacillus paracasei* | Nonpathogenic (part of normal flora beneficial organism needed by human body); shows antiproliferative effect of milk fermented by *L. paracasei* on the growth of a human breast cancer cell line (Biffi et al. 1997); | Biffi et al. 1997, Nutr Cancer 28:93-99 |
| *Lactobacillus plantarum* | Nonpathogenic (part of normal flora beneficial organism needed by human body); Murosaki et al. (2000) described that "that daily administration of *L. plantarum* L-137 is required to exert an antitumor effect at the late stages of tumor development when IL-12 production is considerably impaired"; | Murosaki et al. 2000, Cancer Immunol Immunother 49:157-164 |
| *Lactobacillus rhamnosus* | Nonpathogenic (part of normal flora beneficial organism needed by human body); | |
| *Lactobacillus salivarius* | Nonpathogenic (part of normal flora beneficial organism needed by human body); modification of enteric flora in IL-10 knockout mice by probiotic lactobacilli (through milk feeding) was associated with reduced prevalence of colon cancer and mucosal inflammatory activity (O'Mahony et al. 2001); | O'Mahony et al. 2001, Aliment Pharmacol Ther 15:1219-1225 |
| *Lactobacillus sporogenes* | Nonpathogenic (part of normal flora beneficial organism needed by human body); | |
| *Lactobacillus lactis* | Nonpathogenic (part of normal flora beneficial organism needed by human body); | |
| *Lactobacillus fermentum* | ATCC#9338 | |
| *Streptococcus thermophilus* | Yogurt bacteria; nonpathogenic (part of normal flora beneficial organism needed by human body); reported tumor-specific transplantation resistance in mice after treatment of initial tumors with *S*. *thermophilus* (Kaklij and Kelkar 1996); T-lymphocytes are involved in antitumor activity exhibited by *S. thermophilus* (Kaklij et al. 1991); intraperitoneal administration of *S*. *thermophilus* resulted in complete cure in a very significant proportion of ascitic form of sarcoma-180 or Ehrlich ascites carcinoma tumor-bearing mice (Kelkar et al. 1988); ATCC# BAA-250D. | Kelkar et al. 1988, Cancer Lett 42:73-77; Kaklij et al. 1991, Cancer Lett 56:37-43; Kaklij and Kelkar 1996, Microbiol Immunol 40:55-58 |
| *Bacillus subtilis* | Nonpathogenic; *B. subtilis* bacteremia has been significantly linked with cancer patients (Banerjee et al. 1988); | Banerjee et al. 1988, Arch Intern Med 148:1769-1774 |
| *Bacillus megaterium* | Nonpathogenic; peptidoglycan from *B. megaterium* inhibits tumor growth (Nauciel and Goguel 1977); | Nauciel and Goguel 1977, J Natl Cancer Inst 59:1723-1726 |
| *Bacillus polymyxa* | Nonpathogenic; | |
| *Mycobacterium smegmatis* | Nonpathogenic; fast-growing; Saito and Watanabe (1981) showed that "the bacteriocin from *M. smegmatis* produced morphological alterations and inhibition of synthesis of ribonucleic acid, deoxyribonucleic acid and protein in the transformed but not in the nontransformed cells"; | Lamensans et al. 1975, Proc Natl Acad Sci USA 72:3656-3660; Saito and Watanabe 1981, Microbiol Immunol 25:13-22 |
| *Mycobacterium vaccae* | Nonpathogenic; | |
| *Mycobacterium microti* | Nonpathogenic; ATCC# 35782 | |
| *Mycobacterium habana* | Nonpathogenic; slow-growing, photochromogen originially isolated from monkeys; | |
| *Listeria monocytogenes* | Intracellular pathogen | |
| *Enterococcus faecalis* | Have been isolated from tumors and infection endocarditis; ATCC# 29212, 51299 | Milbrandt 1998, Clin Cardiol 21:123-126 |
| | | |
| | | |
| Aerobes, gram negative | | |
| *Escherichia coli* | Intravenously injected *E*. *coli* shows tumor-specific localization. | Shabahang et al. unpublished data |
| *Salmonella typhimurium* | Bermudes and associates have used intravenously injected *Salmonella* as a protein delivery vector (Pawelek et al. 1997; Bermudes et al. 2000; Clairmont et al. 2000; Zheng et al. 2000;)Bermudes and associates showed a *Salmonella* (TK)-dependent [(14)C]FLAU accumulation of at least 30-fold higher in tumor tissue compared to muscle tissue (Tjuvajev et al. 2001); Phase I study of the intravenous administration of attenuated *Salmonella typhimurium* to patients with metastatic melanoma showed NO ANTITUMOR effect (Toso et al. 2002) | Pawelek et al. 1997, Cancer Res 57:4537-4544; Bermudes et al. 2000, Adv Exp Med Biol 465:57-63; Clairmont et al. 2000, J Infect Dis 181:1996-2002; Zheng et al. 2000, Oncol Res 12:127-135; Tjuvajev et al. 2001, J Control Release 74:313-315; Toso et al. 2002, J Clin Oncol 20:142-152 |
| *Vibrio cholera* | Intravenously injected *V. cholera* shows tumor-specific localization; nonpathogenic strains of *V. cholera* are available; | Shabahang et al. unpublished data |
| *Vibrio harveyi* | Nonpathogenic; luminescent bacteria; ATCC# 700106. | |
| *Pseudomonas fluorescens* | Nonpathogenic; motile by means of multiple polar flagella; *P. fluorescens* bacteremia has been reported in cancer patients (Hsueh et al. 1998); *P. fluorescens* can bind to nerve cells and behave as a pathogen (Picot et al. 2001); normally grow at 26-30°C, but ATCC# 17583 can grow at 37°C. | Hsueh et al. 1998, J Clin Microbiol 36:2914-2917; Picot et al. 2001, Microbes Infect 3:985-995 |
| *Pseudomonas putida* | *P. putida* bacteremia has been reported in cancer patients (Martino et al. 1996); L-Methioninase from *P. putida* depletes methionine and inhibits tumor growth (Kokkinakis et al. 1997); normally grow at 26°C, but ATCC# 43142, 47054 can grow at 37°C. | Pekhov et al. 1983, Biull Eksp Biol Med 95:87-88; Anaissie et al.1987, Am J Med 82:1191-1194; Martino et al. 1996, Eur J Clin Microbiol Infect Dis 15:610-615; Kokkinakis et al. 1997, Nutr Cancer 29:195-204; Miki et al. 2000, Cancer Res 60:2696-2702 |
| | | |
| **Anaerobes, gram positive** | | |
| *Bifidobacterium bifidum* | Nonpathogenic (part of normal flora beneficial organism needed by human body); showed by Kimura et al. (1980) that "selectively localized and proliferated in several types of mouse tumors following i.v. administration" and "None of the same bacilli could be detected in the tissues of healthy organs such as the liver, spleen, kidney, lung, blood, bone marrow, and muscle 48 or 96 hr after i.v. administration into tumor-bearing mice"; shows antiproliferative effect of milk fermented by *B. bifidum* on the growth of a human breast cancer cell line (Biffi et al. 1997); ATCC# 11863, 15696 | Kimura et al. 1980, Cancer Res 40:2061-2068; Biffi et al. 1997, Nutr Cancer 28:93-99 |
| *Bifidobacterium longum* | Nonpathogenic (part of normal flora beneficial organism needed by human body); inhibitory effect on liver tumorigenesis (Mizutani and Mitsuoka 1980); *B. longum* was shown to "selectively localized to and proliferated in 7,12-dimethylbenz[a]anthracene-induced rat mammary tumors after systemic application." (Yazawa et al. 2001); was shown to selectively localize and grow in hypoxic tumors (Yazawa et al. 2000); ATCC# 15707 | Mizutani and Mitsuoka 1980, Cancer Lett 11:89-95; Yazawa et al. 2000, Cancer Gene Ther 7:269-274; Yazawa et al. 2001, Breast Cancer Res Treat 66:165-170 |
| *Bifidobacterium infantis* | Nonpathogenic (part of normal flora beneficial organism needed by human body); shows strong antiproliferative effect of milk fermented by *B. infantis* on the growth of a human breast cancer cell line (Biffi et al. 1997) and other tumor cells or tumors (Kohwi et al. 1978; Sekine et al. 1985; Sekine et al. 1995); ATCC# 15697 | Kohwi et al. 1978, Gann 69:613-618; Sekine et al. 1985, Cancer Res 45:1300-1307; Sekine et al. 1995, Biol Pharm Bull 18:148-153; Biffi et al. 1997, Nutr Cancer 28:93-99 |
| *Bifidobacterium latero-sporus* | Nonpathogenic (part of normal flora beneficial organism needed by human body); | |
| *Bifidobacterium animalis* | Shows antiproliferative effect of milk fermented by *B*. *animalis* on the growth of a human breast cancer cell line (Biffi et al. 1997); ATCC# 25527 | Biffi et al. 1997, Nutr Cancer 28:93-99 |
| *Actinomyces israelii* | Actinomycetes are fungus-like bacteria that form filamentous branches. They are known to reside in the mouth and in the intestinal tract. Pathogenic proliferation of the organisms, which is usually a result of trauma to the region of infection, can lead to actinomycosis. | |
| *Eubacterium lentum* | *Eubacterium* species are normal flora of the intestinal tract. However, they may cause opportunistic infections. *E. lentum,* the most often isolated species, has been linked to endocarditis and some wound infections. | |
| *Peptostrepto-coccus anaerobius* | One of the most common bacteria found in non-sparing anaerobic (NSA) infections in certain surgical group of patients; ATCC# 27337 | Chatterjee and Chakraborti 1995, J Indian Med Assoc 93:333-5, 339 |
| *Peptococcus prevotii* | Found in wound infections; | Chatterjee and Chakraborti 1995, J Indian Med Assoc 93:333-5, 339 |
| *Clostridium novyi* | Strict anaerobe; wildly in soil; motility is inhibited in the presence of oxygen; Vogelstein lab (Dang et al. 2001) showed that "intravenously injected *C*. *novyi-*NT spores germinated within the avascular regions of tumors in mice and destroyed surrounding viable tumor cells", "Large numbers (up to 10⁸ in a volume of 500 µl) of *C*. *novyi* and *C. sordellii* spores could be injected intravenously into normal mice without causing any noticeable side effects."; ATCC# 19402 | Dang et al. 2001, Proc Natl Acad Sci USA 98:15155-15160; Nuyts et al. 2002, Anti-cancer Drugs13:115-125 |
| *Clostridium sordellii* | ATCC#9714 | |
| *Clostridium absonum* | ATCC# 27555 | |
| *Clostridium acetobutylicum* | Theys et al. (2001) demonstrated "Specific targeting of cytosine deaminase to solid tumors by engineered Clostridium acetobutylicum"; Other related papers (Theys et al. 1999); ATCC# 824 | Theys et al. 1999, Appl Environ Microbiol 65:4295-4300; Theys et al. 2001, Cancer Gene Ther 8:294-297 |
| *Clostridium bifermentans* | ATCC# 17836 | |
| *Clostridium difficile* | The link between *C. difficile* and cancer patients has been documented (Anand and Glatt 1993; Simon et al. 2000); ATCC# 700057 | Anand and Glatt 1993, Clin Infect Dis 17:109-113; Schuller et al. 1995, Arch Dis Child 72:219-222; Wehl et al. 1999, Med Pediatr Oncol 32:336-343; Simon et al. 2000, Infect Control Hosp Epidemiol 21:592-596 |
| *Clostridium histolyticum* | ATCC# 19401 | |
| *Clostridium perfringens* | Pathogenic; *C. perfringens* bacteremia has been often reported in cancer patients; *C. perfringens* enterotoxin kills tumor cells and reduce tumor growth (Michl et al. 2001); ATCC# 3624, 13124 | Bodey et al. 1991, Cancer 67:1928-1942; Michl et al. 2001, Gastroenterology 121:678-684 |
| *Clostridium beijerinckii* | Brown and associates at Stanford described that "To demonstrate the specificity of this approach for tumor targeting, we intravenously injected the inactive spore form of *C. beijerinckii,* which upon transition to a reproductive state will express the E. *coli* nitroreductase gene. Nitroreductase activity was detectable in 10 of 10 tumors during the first 5 days after intravenous injection of inactive clostridial spores, indicating a rapid transition from spore to reproductive state" (Lemmon et al. 1997); other related papers on *C*. *beijerinckii* tumor targeting (Minton et al. 1995, FEMS Microbiol Rev 17:357-364; Fox et al. 1996); ATCC# 25752 | Minton et al. 1995, FEMS Microbiol Rev17:357-364; Fox et al. 1996, Gene Ther 3:173-178; Lemmon et al. 1997, Gene Ther 4:791-796 |
| *Clostridium sporogenes* | Harmless saprophyte; Brown and associates at Stanford described that "systemic delivery of 5-FC into mice previously injected with CD-transformed spores of C. *sporogenes* produced greater antitumor effect than maximally tolerated doses of 5-FU"; ATCC# 19404 | Liu et al. 2002, Gene Ther 9:291-296 |
| *Staphylococcus aureus* | Non-motile, non-sporing and facultatively anaerobic; a halotolerant (salt tolerant) organism associated with the nasal mucosa of mammals which has both benign and pathogenic strains; *S*. *aureus* bacteremia is frequently seen in cancer patients; ATCC# 25923 | |
| *Staphylococcus epidermidis* | Nonpathogenic normal microflora component of the skin; | |
| | | |
| | | |
| **Anaerobes, gram negative** | | |
| *Acidaminococcus fermentans* | Found in wound infections; | Chatterjee and Chakraborti 1995, J Indian Med Assoc 93:333-5, 339 |
| | | |
| | | |
| **Plant bacteria, gram positive** | | |
| *Clavibacter michiganensis* subsp. *michiganensis* | Pathogen of tomato | |
| | | |
| | | |
| **Plant bacteria, gram negative** | | |
| *Agrobacterium tumefaciens* | *A. tumefaciens* in plant tumors (Ullrich and Aloni 2000); Kunik et al. (2001, Proc Natl Acad Sci U S A 98:1871-1876) described "*Agrobacterium* attaches to and genetically transforms several types of human cells"; it has been reported that antibodies against *A. tumefaciens* were found in patients with various cancers (Aksac 1974, Turk Hij Tecr Biyol Derg 34:48-51); grow at 26-30°C; ATCC# 15955 | Aksac 1974, Turk Hij Tecr Biyol Derg 34:48-51; Zambryski et al. 1982, J Mol Appl Genet 1:361-370; Rezmer et al. 1999, Planta 209:399-405; Ullrich and Aloni 2000, J Exp Bot 51:1951-1960; Azmi et al. 2001, Planta 2001 May;213(1): 29-36; Kunik et al. 2001, Proc Natl Acad Sci USA 98:1871-1876 |
| *Erwinia herbicola* | Non-capsulated, non-spore forming, short rods with a single monotrichous polar flagellum, and are harmless to humans; purified tyrosine phenol-lyase from *E. herbicola* significantly inhibited growth of established B-16 melanoma tumors; | Meadows et al. 1976, Cancer Res 36:167-167 |
| *Azorhizobium caulinodans* | Symbiotic, colonize plants, fix nitrogen; | |
| *Xanthomonas campestris* pv. *vesicatoria* | Pathogen of pepper and tomato | |
| *Xanthomonas campestris* pv. *campestris* | Pathogen of beat and cabbage; *E*. *coli lac* promoter was shown to be functional in this plant pathogen; | Soby and Daniels 1996, Appl Microbiol Biotechnol 46:559-561 |
| | | |

| | | |
|---|---|---|
| **Note: The content of this table is by no-means to be exhaustive. Any other similar bacterial strains, which are not listed in this table, are also considered to be included.** | | |

A further example of a bacterium useful for the preparation of a diagnostic composition for tumor-imaging or monitoring a therapeutic tumor treatment is a magnetic bacterium (Metal-Binding-Bacteria-Mediated Tumor Detection (MBBMTD)). Such bacteria allow tumor detection through the accumulation of iron-based contrast agents. Magnetic bacteria can be isolated from fresh and marine sediments. They are capable of producing magnetic particles (Fe₃O₄) (Blakemore, Annu. Rev. Microbiol. 36 (1982), 217-238). To do so, they have efficient iron uptake systems, which allow them to utilize both insoluble and soluble forms of iron. *Magnetospirillum magneticum* AMB-1 is an example of such magnetic bacteria that has been isolated and cultured for magnetic particle production (Yang et al., Enzyme Microb. Technol. 29 (2001), 13-19). Since it can be expected that these magnetic bacteria (naturally occuring or genetically modified), when injected intravenously, possess the similar tumor accumulation ability as that of vibrio *cholera,* for example, these bacteria can be used for accumulating iron-based contrast agents in the tumors, which in turn allows tumor detection by MRI. Similarly, other naturally isolated metal-accumulating strains of bacteria can be used for tumor targeting, absorption of metals from contrast agents, and eventually tumor imaging.

An LIVP strain of *Vaccinia* virus is used for the diagnostic and therapeutic compositions used in the present invention; see Table 2 listing examples of viruses useful for the same purpose as the present invention.

**Table 2. Examples of viruses useful for the same purpose as the present invention:**

| **Viral strains** | **Brief description** | **References** |
|---|---|---|
| *Vaccinia* virus | We have shown that viral thymidine kinase mutation is not necessary for tumor accumulation by intravenously injected *Vaccinia* virus. | Yu et al. 2002, unpublished data |
| *Epstein-Barr virus* | | |
| *Human papillomavirus* | | zur Hausen 2002, Nat Rev Cancer 2:342-350 |
| *Retrovirus* | | |
| *Adenovirus* | | Lindsey 2002, Lancet Oncol 3:264 |
| *Adenoassociated virus* | | |
| *SV40 virus* | | |
| *Cytomegalovirus* | | |
| *Newcastle Disease Virus* | safe, replicates in and kill tumor cells; local injection is more effective than systemic injection | Schirrmacher et al. 2001, Int J Oncol 18:945-952 |
| *Bovine enterovirus* | Bovine enterovirus has been shown to exhibit wide tissue tropism for cell types *in vitro.* It also shows oncolytic activity towards human cells. | Smyth et al. 2002, Int J Mol Med 10:49-53 |
| *Lymphocytic choriomeningitis virus (LCMV)* | high stability, low toxicity, and broad host range | Beyer et ad. 2002, J Virol 76:1488-1495 |
| *Lentiviruses* | | |
| *Derivatives of the Edmonston-B strain of measles virus (MV-Ed)* | safe, live attenuated, has been shown to induce regression of human lymphoma xenografts in immunodeficient mice | Grote et al. 2001, Blood 97:3746-3754 |
| *Herpes simplex virus type* I | | Lachmann and Efstathiou 1999, Curr Opin Mol Ther 1:622-632; |
| | | Wu et al. 2001, Cancer Res 61:3009-3015 |
| *Attenuated yellow fever (YF) virus* | powerful vaccine, safe | Barrett 1997, Biologicals 25:17-25; |
| | | McAllister et al. 2000, J Virol 74:9197-205 |

| | | |
|---|---|---|
| **Note: The content of this table is by no-means to be exhaustive. Any other similar viral strains, which are not listed in this table, are also considered to be included.** | | |

In the invention, the virus is an LIVP strain of *Vaccinia* virus.

A mammalian cell such as stem cells which can be autologous or heterologous to the organism is suitable for a diagnostic or therapeutic composition for the same purpose as the present invention. Examples of suitable cell types are shown in Table 3.

**Table 3. Examples of mammalian cells useful for the same purpose as the present invention:**

| **Mammalian cells** | **Brief description** | **Reference** |
|---|---|---|
| Stem cells | It has been shown that neural stem cells implanted intravenously outside the central nervous system target to an intracranial tumor. Additionally, when implanted intracranially at distant sites from the tumor, such as into the contralateral hemisphere or into the cerebral ventricles, the donor neural stem cells migrate through normal brain tissues to target the human glioblastoma cells. | Aboody et al. 2000, Proc Natl Acad Sci U S A 97:12846-12851; |
| | | Herrlinger et al. 2000, Mol Ther 1:347-357 |
| Different types of tumor cells | For example, ovarian cancer cells have been shown to infiltrate the pleural space of patients to target malignant pleural mesotheliomas (Harrison et al. 2000, Ann Thorac Surg 70:407-411). We have shown that intravenously injected fibrosarcoma cells accumulate in breast tumors and subcutaneous glioma tumors (Yu et al. unpublished data). | |

| | | |
|---|---|---|
| **Note: The content of this table is by no-means to be exhaustive. Any other similar types of mammalian cells, which are not listed in this table, are also considered to be included.** | | |

In a further preferred embodiment of the diagnostic and/or therapeutic composition of the present invention the luminescent or fluorescent protein is a luciferase, green fluorescent protein (GFP) or red fluorescent protein (RFP).

In a particularly preferred embodiment, the LIVP strain of *Vaccinia* virus of the diagnostic and/or pharmaceutical composition of the present invention additionally contains a gene encoding a substrate for the luciferase. In an even more preferred embodiment, the LIVP strain of *Vaccinia* virus of the diagnostic and/or pharmaceutical composition of the present invention contains a *ruc-gfp* expression cassette which contains the *Renilla* luciferase *(ruc)* and *Aequorea gfp* cDNA sequences under the control of a strong synthetic early/late (PE/L) promoter of *Vaccinia* or the *luxCDABE cassette.*

A preferred use of the virus described above is the preparation of a diagnostic composition for tumor-imaging. The diagnostic composition of the present invention can be used e.g. during surgery, to identify tumors and metastasis. Furthermore, the diagnostic composition of the present invention is useful for monitoring a therapeutic tumor treatment. Suitable devices for analysing, e.g., the localization or distribution of luminescent and/or fluorescent proteins in an organism, organ or tissue are well known to the person skilled in the art and, furthermore described in the literature cited above as well as the examples, below. Additionally, the virus can be modified in such a way that they bind metals and consequently are useful in MRI technology to make tumor localization more specific.

The present invention also relates to the use of an antimicrobial, e.g. antibacterial or antiviral compound, e.g., peptide or protein fused to a detectable protein for the preparation of a diagnostic composition for tumor-imaging or monitoring a therapeutic tumor treatment. This diagnostic composition is useful for tumor detection through the binding of the antimicrobial compound, e.g., a light-emitting or radiolabeled antimicrobial peptide/protein with bacteria localized in tumors (Peptide-Linked-Tumor-Targeting-Vector-Detection (FLTTVD)).

The present invention also relates to the use of an antimicrobial, e.g. antibacterial or antiviral compound, e.g., peptide or protein fused to a therapeutic protein for the preparation of a pharmaceutical composition for tumor therapy.

Examples of naturally occurring antimicrobial proteins include ubiquicidin (UBI, 6.7 kDa) and lactoferrin (hLF, 77 kDa). UBI was originally isolated from murine macrophages and later from human airway epithelial cells. hLF is found in mucosa and neutrophils, and is known to bind to surface structures of both gram-negative and gram-positive bacteria. Small synthetic peptides containing the bacteria binding domains of UBI or hLF have been designed for imaging of bacterial infections, which can be discriminated from sterile inflammations (Nibbering et al., Nucl. Med. Commun. 19 (1998), 1117-1121; Welling et al., Nucl. Med. Biol. 29 (2000), 413-422; Welling et al., Eur. J. Nucl. Med. 27 (2002), 2929-301). These peptides are radiolabeled with ^{99m}Tc, which allows real-time visualization in live animals using planar scintigraphy. For example, a planar gamma camera (e.g. Toshiba GCA 7100/UI, Tokyo, Japan) equipped with a low-energy general-purpose parallel-hole collimator can be used to visualize the distribution of ^{99m}TC-labelled antimicrobial peptides in live animals or humans. To apply these synthetic peptides for tumor detection, first the tumorous individuals are infected with a particular strain of extracellular bacteria. After bacterial colonization of the tumors, the radiolabelled compounds, e.g., peptides are delivered intravenously. Specific binding of the labeled peptides to the bacteria in the tumors can be visualized in real time by scintigraphy, which therefore allows the localization of tumors in humans and animals. In addition to labeling with ^{99m}TC, the compounds, e.g., synthetic peptides can also be labeled with paramagnetic- or superparamagnetic-metal particles for visualization using MRI or the compounds can be labeled with radionuclides, such as ⁵⁵CO, ⁶⁸Ga, ⁶⁰CU(II), ¹²³I etc. for non-invasive visualization by SPECT or PET. Furthermore, the compounds, e.g., synthetic peptides can also be tagged with fluorescent probes, fluorescent proteins (such as green fluorescent protein (GFP) or DsRed), or luminescent proteins (such as *Renilla* luciferase, firefly luciferase) for real-time visualization in an individual.

In addition to UBI and hLF, there are many other examples of antimicrobial peptides/proteins produced by bacteria, plants, invertebrates, and vertebrates including humans (Schroder, Cell. Mol. Life Sci. 56 (1999), 32-46; Cole and Ganz, Biotechniques 29 (2000), 822-826, 828, 830-831) which are useful for the purposes of the present invention, e.g. pediocin PA-1 from lactic acid bacteria, gramicidin S from *Bacillus brevis*), protegrin-1 from porcine leukocytes, SMAP-29 from sheep myeloid cells, buforin I & buforin II from Asian toad, beta-defensins, LL-37, a fragment of human cathelicidin hCAP-18, arasin I from catfish, granulysin, PAMP from *Propionibaeterium jensenii* etc. Based on these naturally occurring antimicrobial proteins, small peptides can be designed and synthesized to retain the bacteria-binding capability while eliminating the bacteria-killing effect by the original proteins. These synthetic peptides could then be used in bacterium-mediated tumor detection.

Similar to detection of bacteria by antibacterial compounds (e.g. peptides/proteins), antiviral compounds (e.g. peptides/proteins) may be used for detecting viral particles in the tumors. Lactoferricin is one of the examples of antiviral peptide/protein that may be used to design peptides for detecting viral particles. In addition to the antimicrobial peptides/proteins discussed, radiolabelled- or light-emitting-protein/probe-labeled antibody fragments can also be used to target bacteria or viruses localized in tumors for tumor detection.

Moreover, the antimicrobial compound can be fused to a metal-binding protein (Fusion-Peptide-Linked Tumor Targeting Vector Detection (PLTTVD)) for detecting the accumulation of bacteria, viruses etc. according to the present invention in tumors. After allowing the binding of the fusion construct to the engineered bacteria etc. in tumor, metal agents (which can be used for detection by MRI, PET or SFECT) are injected intravenously. Binding and absorption of the metal agents by the bacteria etc. will allow indirect tumor detection.

The present invention also relates to a pharmaceutical composition comprising an LIVP strain of *Vaccinia* virus containing DNA sequence(s) encoding a cell receptor capable of binding a ligand and furthermore a ligand which is coupled to a toxin (= chimeric toxin). Suitable toxins which can be coupled to the ligand are well known to the person skilled in the art. Several chimeric toxins have been described previously. First, the Tfn-CRM 107 chimeric toxin was prepared by conjugating transferrin to the mutated diphtheria toxin CRM 107 (Johnson et al., J. Neurosurg. 70 (1989), 240-248), which targets the transferrin receptor. Second, the 425.3-PE immunotoxin was prepared by conjugating anti-EGF receptor antibody to the whole Pseudomonas exotoxin A (Myklebust et al., Cancer Res. 53 (1993), 3784-3788). Third, the Tfn-PE was prepared by conjugating transferrin to the Pseudomonas exotoxin (Hall et al., J. Neurosurg. 76 (1992), 838-844; Hall et al., Neurosurgery 34 (1994), 649-656). It could be shown in animal models and human patients that these chimeric toxins have anti-tumor activities against medulloblastoma, small cell lung cancer, glioma xenografts and intracranial tumors. Thus, e.g. recombinant *Vaccinia* virus can be designed to specifically express transferrin receptors on the surface of tumor cells allowing the use of these receptor-targeting chimeric toxins for antitumor treatment.

The present invention also relates to a pharmaceutical composition comprising (a) an LIVP strain of *Vaccinia* virus containing a DNA sequence encoding a cell receptor capable of binding a ligand and (b) said ligand, which is coupled to a therapeutic protein. Suitable therapeutic proteins as well as methods for generating therapeutic ligand fusion proteins by linking therapeutic proteins to the ligand proteins are well known to the person skilled in the art. Examples of suitable therapeutic proteins are shown in Tables 4 and 5.

**Table 4. Examples of enzyme/prodrug pairs in which the delivery of the enzyme gene is facilitated by intravenously injected microorganisms and cells:**

| **Therapeutic proteins (enzymes), drugs, prodrugs** | **Description** | **References** |
|---|---|---|
| Herpes simplex virus thymidine kinase (HSV-TK) | Most well-known enzyme/prodrug combination. | Moolten Cancer Res. 1986;46:5276-5281 |
| + Ganciclovir (GCV) | | |
| Herpes simplex virus thymidine kinase (HSV-TK) | A-5021 has highly selective anti-hepatic activity and was selectively phosphorylated by viral TK in herpes virus-infected cells. The anti-herpetic activity of A-5021 was most potent in comparison with ACV and penciclovir. | Hasegawa et al. 2000, Cancer Gene Ther 7:557-562 |
| + A-5021 (1'S,2'R)-9{[1',2'-bis(hydroxymethyl)cyclopr op-1'-yl]methyl}guanine | | |
| Horseradish peroxidase (HRP) | When activated by purified HRP, IAA was shown to inhibit colony formation in mammalian cells, whereas, neither enzyme nor prodrug alone was cytotoxic at the same concentration or times. | Greco et al. 2000, Cancer Gene Thera. 7:1414-1420 |
| + Indole-3-acetic acid (IAA) | | |
| | | |
| | The HRP/IAA-induced cell kill was effective in normoxic and anoxic conditions. | |
| Bacterial enzyme carboxy-peptidase G2 (CPG2) | CPG2 can be expressed both intracellularly or tethered to the outer surface of mammalian cells, where it is able to activate mustard prodrugs for use in suicide gene therapy. | Spooner et al. 2000, Cancer Gene Ther. 7:1348-1356 |
| + 4-([2-chloroethyl][2-mesyloxyethyl] amino)benzoyl-L-glutamic acid (CMDA) | | Webley et al. 2001, Br J Cancer 84:1671-1676 |
| or + 4-[N,N-bis(2-iodoethyl) amino] phenoxycarbonyl L -glutamic acid (ZD2767P) | | |
| Human cytochrome P450 CYP lA2 | Acetaminophen is cytotoxic through the cytochrome P450-mediated generation of a chemically reactive metabolite, N-acetylbenzoquinoneimine (NABQI). | Thatcher et al. 2000, Cancer Gene Ther 7:521-525 |
| + acetaminophen | | |
| Rabbit cytochrome P450 4B1 (CYP4B1) | CYP4B1 is able to induce tumor cell death at low micro-molar concentrations in glioblastoma cells after treatment with the prodrug 4-IM. | Mohr et al. 2000, Cancer Gene Ther. 7:1008-1014; |
| + 4-ipomeanol (4-IM) | | Heuser et al. 2000, Cancer Gene Ther 7:806-12 |
| Rat cytochrome P450 4B 1 (CYP2B1) | The CYP2B1 gene product activates oxaphosphorines to the hydroxy form, giving rise to the toxic products phosphamide mustard and acrolein, which alkylate DNA and proteins, respectively. | Kammertoens et al. 2000, Cancer Gene Ther 7:629-636 |
| + oxaphosporines, such as ifosfamide (IFO) | | |
| *E. coli* nitroreductase (NTR) | CB1954 is a weak monofunctional alkylating agent that is converted by NTR into 2- and 4-hydroxylamino derivatives. Cellular thioesters such as acetyl coenzyme A subsequently convert the latter into a powerful bifunctional alkylating agent that can kill both proliferating and nonproliferating cells. | Djeha et al. 2000, Cancer Gene Ther. 7:721-731; |
| + CB1954 | | Djeha et al. 2001, Mol Ther 3:233-240 |
| | | |
| | PTX0147 is the plasmid expressing NTR from the human cytomegalovirus (CMV) early promoter/enhancer and also carries the b-globin second intron and poly (A) sequences and a G418 selectable marker. | Westphal et al. 2000, Cancer Gene Ther 7:97-106 |
| | | Weedon et al. 2000, Int J Cancer 86:848-854 |
| *E. coli* cytosine deaminase (CD), E. *coli* uracil phosphoribosyltransferase (UPRT) | Despite CD expression, a number of tumor cells were 5-FC-resistant, which may be attributable to the lack of an active cytosine transport | Koyama et al. 2000, Cancer Gene Ther. 7:1015-1022; |
| | | Theys et al. 2001, Cancer Gene Ther 8:294-297; |
| + 5-fluorocytosine (5-FC) | system in mammalian cells and to the degradation of the formed 50FU by dihydropyrimidine dehydrogenase (DPD). In the gene transfer strategy, to improve the effect of the CD/5-FC system, it might be possible to transduce the enzyme gene that converts 5-FU to its active forms. One of the candidates is *E. coli* UPRT. It is a pyrimidine salvage enzyme and is characteristic to bacteria. It directly converts 5-FU to 5-fluorouridine monophosphate (FUMP) at the first step of 5-FU activation and has the potential to enhance the activating pathway against DPD. | Kammertoens et al. 2000, Cancer Gene Ther 7:629-636; Block et al. 2000, Cancer Gene Ther 7:438-445; Bentires-Alj et al. 2000 Cancer Gene Ther 7:20-26; Kawamura et al. 2000, Cancer Gene Ther 7:637-643; Li et al. 1997, Cancer Gene Ther 4:113-117 |
| Cytochrome P450 enzymes | Liver tissue has a high content of P450 enzymes active toward | Huang et al. 2000, Cancer Gene Ther. 7:1034-1042; |
| + cyclophosphamide (CPA) | CPA and is the major organ responsible for CPA activation. Activated CPA generated in the liver circulates through the blood and gains entry to tumor tissue to exert its therapeutic effects. | Kan et al. 2001, Cancer Gene Ther 8:473-482 |
| | | |
| | Intratumoral CPA activation can result in a high, localized concentration of active drug metabolite at its site of action, which may maximize therapeutic effects while at the same time minimizing the host toxicities associated with hepatic drug activation. | |
| rabbit carboxylesterase | replication-deficient AdRSVrCE for 24 h and subsequent exposure of cells to 1-5 microM CPT-11 for 4 h increased the toxicity of CPT-11 to three | Meck et al. 2001, Cancer Res61:5083-5089 |
| + 7-ethyl-10-[4-(1-piperi-dino)-1-piperidino]carbonyloxycamp-tothecin (CPT-11) | Neuroblastoma cell lines (SJNB-1, NB-1691, and SK-N-SH) from approximately 20-50-fold and eradicated their clonogenic potential. | |
| Mushroom tyrosinase | A sterically undemanding prodrug bis-(2-chloro-ethyl)amino-4-hydroxyphenylaminomethanone 28 was synthesised and found to be oxidised by mushroom tyrosinase at a superior rate to tyrosine methyl ester, the carboxylic acid of which is the natural substrate for tyrosinase. | Jordan et al 2001, Bioorg Med Chem 9:1549-1558 |
| + bis-(2-chloroethyl)amino-4-hydroxyphenylaminomethanone 28 | | |
| *E. coli* β-galactosidase | Prodrug cleaved by galactosidase shows high cytotoxicity towards human bronchial carcinoma cells of line A549. | Tietze et al. 2001, Chembiochem 2:758-765 |
| + 1-chloromethyl-5-hydroxy-1,2-dihydro-3H-benz[e]indole (CC-1065) | | |
| or + 1-(1'-chloroethyl)-5-hydroxy-1,2-dihydro-3H-benz[e]indole | | |
| A mutant of carboxypeptidase G2 (CPG2, glutamate carboxypeptidase | Activation of all three of the prodrugs not only kills the cells expressing the mutant CPG2 on the surface but also the neighboring cells through by-stander effect. | Friedlos et al. 2002, Cancer Res 62:1724-1729 |
| + 4-[bis(2-iodoethyl)amino]-phenyloxycarbonyl-L-glutamic acid | | |
| or + 3-fluoro-4-[bis(2-chlorethyl)amino]benzoyl-L-glutamic acid or + 3,5-difluoro-4-[bis(2-iodoethyl)amino]benzoyl-L-glutamic acid | | |

**Table 5. Further examples of therapeutic proteins that can be used for BMPT (bacterium-mediated protein therapy), VMPT (virus-mediated protein therapy) or CMPT (cell-mediated protein therapy), e.g. mCMPT (mammalian-cell-mediated protein therapy), against tumors:**

| **Therapeutic proteins, drugs** | **Description** | **References** |
|---|---|---|
| rsCD40L | Ligand of CD40, sensitizing tumor cells to apoptosis induced by a variety of agents, including TNF-alpha, anti-Fas, and cytotoxic drugs. | Eliopoulos et al. 2000, Mole. Cell. Biol. 20:5503-5515 |
| Fas-ligand | | Sharma et al. 2000, Pharmacol Ther. 88:333-347 |
| TRAIL | Ligand for death receptors such as DcR2, DcR1, DR5, DR4. | Golstein 1997, Curr. Biol. 7:R750-753. |
| TNF | TNF is the ligand for TNFR1, which mediates cell-death signaling. | Baker and Reddy 1996, Oncogene 12:1-9; Theys et al. 1999, Appl Environ Microbiol 65:4295-4300; Lammertyn et al. 1997, Appl Environ Microbiol 63:1808-1813 |
| | | |
| **Recombinant antibodies** | | |
| Anti-GA733-2 | Secreted or membrane-anchored form of monoclonal antibody (mAb) (CO17-1A) specific for Ag (GA733-2) that is present on the surface of most gastrointestinal carcinomas. | Paul et al. 2000, Cancer Gene Ther 7:615-623 |
| Anticancer drugs adriamycin (ADM), cytosine arabinoside (Ara-C), cisplatin, doxorubicin, mitomycin C, fluorouracil, campto-thecin, cis-diammine-dichloroplatinum (II), CDDP, etc. + anti-Fas antibody | Some tumor cells express Fas antigen on their surface, and apoptosis is induced in those cells by IgM-anti-Fas MoAb. Clinically relevant concentrations of anticancer drugs enhance Fas receptor expression on the plasma membrane of tumor cells. By the combination of ADM or Ara-C with IgM-anti-Fas MoAb, the induction of apoptosis in HL60 leukemic cells was enhanced significantly. Therefore, we may use bacteria or viruses to deliver the gene encoding anti-Fas antibody. Expression of the anti-Fas antibody on the surface of tumor cells may sensitizes these cells to chemotherapy agents, which therefore may improve the efficiency of chemotherapy. | Nakamura et al. 1997, Anticancer Res. 17:173-179; Micheau et al. 1997, J. Natl. Cancer Inst. 89:783-789; Chang et al. 1998, Osaka City Med. J. 44:173-180; Mizutani et al. 1997, Cancer 79:1180-1189; Jiang et al. 1999, Hepatology 29:101-110; Mizutani et al. 1998, J. Urol. 160:561-570 |
| Bacterial toxins | | |
| Microcin E492 | A low-molecular mass channel-forming bacteriocin (7,887 Da) produced by *Klebsiella pneumoniae.* It has been show that microcin E492 induces apoptosis in certain human cell lines. Treatment with zVAD-fink, a general caspase inhibitor, completely blocked the cytotoxic effect of this bacteriocin, which may provide a safety mechanism when microcin is used in anti-tumor treatment. Microcin-E492-insensitive mutants of E. coli K12 have been isolated, which may be used as a carrier to deliver microcin (Pugsley et al. 1986, J Gen Microbiol 132:3253-3259). | de Lorenzo 1984, Arch Microbiol 139:72-75; |
| | | Hetz et al. 2002, Proc Natl Acad Sci U S A 99:2696-2701 |
| diphtheria toxin (DT) | Toxin-labeled monoclonal antibodies have been used to target tumor cell surface receptors for cell killing (Kreitman 2001, Curr Pharm Biotechnol 2:313-325; Thomas et al. 2002, J Pediatr Surg 37:539-544). Toxin-label protein ligands have also been used to target cell surface receptors (Olson et al. 1997, Int J Cancer 73:865-870; Arora et al. 1999, Cancer Res 59:183-188; Wild et al. 2000, Br J Cancer 83:1077-1083). | |
| *Pseudomonas* exotoxin | | |
| *Escherichia coli* Shiga toxins | Shiga toxins, Shiga-like toxin I (SLT-1) and Shiga-like toxin II (SLT-II) are cell-associated cytotoxins, which have been shown to be able to kill tumor cells. The toxins kill target cells by inducing apoptosis. | O'Brien et al. 1992, Curr Top Microbiol Immunol 180:65-94; |
| | | Nakao and Takeda 2000, J Nat Toxins 9:299-313 |
| *Escherichia coli Verotoxin I (VT1)* | VT1 is an E. *coli* elaborated subunit toxin active only against (tumor) cell lines that express the VT1 receptor, globotriaosyl ceramide-Gb3. In an example of VT1 action, it has been shown that VT1 eliminates human astrocytoma xenograft in nude mice. | Farkas-Himsley et al. 1995, Proc Natl Acad Sci U S A 92:6996-7000; |
| | | Arab et al. 1999, Oncol Res 11:33-39 |
| | | |
| Plant toxins | | |
| Hyperforin | Hyperforin is a plant derived antibiotic. It was shown that hyperforin can inhibit tumor growth by activating mitochondria-mediated apoptosis pathway. | Hostanska et al. 2002, Pharmazie 57:323-331; |
| | | Schempp et al. 2002, Oncogene 21:1242-1250 |

Since the tumor tissues can be labeled with ligand receptors using intravenously injected engineered bacteria, viruses or cells, the binding of therapeutic-ligand fusion proteins to the ligand receptors will enable the targeting of the therapeutic proteins to the tumor tissues. Intracellular bacteria and viruses are particularly useful in labeling the tumor cell surface with designated receptor proteins. However, due to "bystander effect" of therapeutic proteins on neighboring cells, extracellular bacteria and mammalian cells can also be used to deliver the therapeutic proteins.

In the case of Gene-Directed Enzyme Prodrug Therapy (GDEPT) (see below), for example, extracellular bacteria may be used to secrete the enzyme-ligand fusion protein. The tumor cell surface may be labeled with receptor proteins by viruses. After the intravenous delivery of prodrug, not only the cells expressing the receptors will be bathed with and killed by the active drug, the surrounding tumor cells (which may not be labeled with receptors) will also be killed.

An LIVP strain of *Vaccinia* virus may be used to label the tumor cell surface with receptor proteins. For example, the tumor tissues can be specifically infected by intravenously injected engineered *Vaccinia* virus carrying, e.g., a transferrin receptor gene (also encoding a single peptide for cell surface expression). Expression of the transferrin receptor on the tumor cell surface will mark these cells for targeting by therapeutic-ligand fusion proteins. In this case, the ligand is the transferrin protein, and the therapeutic protein could be a *Pseudomonas* exotoxin (or any other cytotoxic therapeutic proteins). Tumor cell internalization of the transferrin/transferrin receptor pair allows the internalization of the therapeutic protein, which in turn delivers the cytotoxicity specifically to the tumor cells. Transferrin/transferrin receptor pair is only one of many examples of ligand-receptor pairs that can be used. In addition, mutant ligands and mutant receptors with highly specific affinity toward each other may be used to avoid the binding to endogenous proteins.

Additional examples of suitable proteins are human endostatin and the chimeric PE37/TGF-alpha fusion protein. Endostatin is a carboxyterminal peptide of collagen XVIII which has been characterized (Ding et al., PNAS USA 95 (1998), 10443). It has been shown that endostatin inhibits endothelial cell proliferation and migration, induces G1 arrest and apoptosis of endothelial cells *in vitro*, and has antitumor effect in a variety of tumor models. Intravenous or intramuscular injection of viral DNA and cationic liposome-complexed plasmid DNA encoding endostatin result in limited expression levels of endostatin in tumors. However intratumoral injection of purified endostatin shows remarkable inhibition of tumor growth. *Pseudomonas* exotoxin is a bacterial toxin secreted by *Pseudomonas aeruginosa.* PE elicits its cytotoxic effect by inactivating elongation factor 2 (EF-2), which results in blocking of protein synthesis in mammalian cells. Single chain PE is functionally divided into three domains: domain Ia is required for binding to cell surface receptor, domain II is required for translocating the toxin into the target cell cytosol, and domain III is responsible for cytotoxicity by inactivating EF-2. PE40 is derived from wild type *Pseudomonas* exotoxin that lacks the binding domain Ia. Other proteins such as antibody fragments or protein ligands can be inserted in place of the binding domain. This will render the PE40-ligand fusion protein specific to its receptor. One of the highly specific engineered chimeric toxins is the TGF alpha/PE40 fusion protein, where the C-terminus of TGF alpha polypeptide has been fused in frame with the N-terminus of the PE40 protein. TGF alpha is one of the ligands of epidermal growth factor receptor (EGFR), which has been shown to be preferentially expressed on the surface of a variety of tumor cells. TGF alpha-PE40 fusion protein has been shown to be highly toxic to tumor cells with elevated EGFRs on the cell surface and while it is less toxic to nearby cells displaying fewer numbers of surface EGFR. The toxicity of TGF alpha-PE40 chimeric protein is dependent on a proteolytic processing step to convert the chimeric protein into its active form, which is carried out by the target. To overcome the requirement for proteolysis, a new chimeric toxin protein that does not require processing has been constructed by Theuer and coworkers (J. Biol. Chem. 267 (1992), 16872). The novel fusion protein is termed PE37/TGF alpha, which exhibited higher toxicity to tumor cells than the TGF alpha-PE40 fusion protein.

In a particular preferred embodiment of the diagnostic and/or pharmaceutical composition used in the present invention the ligand is not the naturally occurring ligand but any compound which is capable of specifically binding to the receptor, e.g. an antibody. The term "antibody", preferably, relates to antibodies which consist essentially of pooled monoclonal antibodies with different epitopic specificities, as well as distinct monclonal antibody preparations. Monoclonal antibodies are made from an antigen containing fragments of the particular receptor by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab')2 fragments) which are capable of specifically binding to a receptor. Fab and F(ab')2 fragments lack the Fc fragment of intact antibody, clear more rapidly from the circulation, and may have less non-specific tissue binding than an intact antibody (Wahl et al., J. Nucl. Med. 24:316-325 (1983). Thus, these fragments are preferred, as well as the products of an FAB or other immunoglobulin expression library. Moreover, antibodies of the present invention include chimeric, single chain, and humanized antibodies.

Antibody-ligands are, e.g., useful in anti-tumor therapy using fusion proteins (i.e. immunotherapeutic proteins) between antibodies and therapeutic proteins: Firstly, the genes encoding cell surface receptors are delivered to tumors cells in live organisms via intravenously injected engineered bacteria and viruses. Such receptors can be, e.g., transferrin receptor, EGF receptor, somatostatin receptor, etc. Over-expression of receptors on the cell surface following bacterial or viral infections will be used to mark the tumor cells. Secondly, a fusion protein (an immunotherapeutic protein) between an antibody, preferably an antibody fragment, (specific to the overexpressed surface receptors) and a therapeutic protein (e.g. any type of toxins, see Table 5) is prepared. The intravenously injected fusion protein may bind to the marked tumor cells and exhibit cytotoxicity toward these cells in the tumor tissues.

The present invention also relates to the use of a diagnostic and/or pharmaceutical composition containing an LIVP strain of *Vaccinia* virus as described above and furthermore (a) an antimicrobial compound fused to a protein suitable for tumor therapy and/or elimination of metastatic tumors and/or (b) an antimicrobial compound fused to a detectable protein or a protein capable of inducing a detectable signal. Anti-tumor therapy using such fusion proteins between antimicrobial compounds (e.g. peptides/proteins) and therapeutic proteins can be carried by firstly preparing fusion gene constructs encoding hybrid proteins between antimicrobial peptides/proteins and therapeutic proteins (see Table 5, above). After protein expression, the hybrid proteins are purified and ready for intravenous injection. Secondly, e.g. light-emitting bacteria, which can be recognized and bound by the antimicrobial peptides/proteins, are injected intravenously into the subject for tumor-specific accumulation. The specific binding of antimicrobial peptides/proteins to bacteria in the tumors helps to concentrate therapeutic proteins specifically in the tumors, which therefore may elicit tumor specific cytotoxicity.

Furthermore, the protein suitable for tumor therapy and/or elimination of metastatic tumors can be an enzyme converting an inactive substance (prodrug) administered to the organism into an active substance, i.e. toxin, which kills the tumor or metastasis (Gene-Directed Enzyme Prodrug Therapy (GDEPT)). The principle of GDEPT is that an enzyme/protein activates a systemically delivered nontoxic prodrug into active toxic drug, which is cytotoxic to tumors. To be specific, GDEPT is a two-step treatment. In the first step, the gene encoding a foreign enzyme is administered and directed to the tumor, where it may be expressed using specific transcriptional elements. In the second step, prodrugs are administered and activated by the foreign enzyme expressed at the tumor. If the enzyme/proteins are present only in the tumors, the active drugs will also be produced only in the tumors, and therefore exhibit cytotoxicity only toward the tumor cells, while at the same time, systemic toxicity is kept to a minimum. The gene encoding enzyme/protein is delivered specifically to the tumors using intravenously injected engineered virus according to the present invention. These genes can be under the control of either constitutive promoters or exogenously regulated inducible promoters additionally ensuring that the conversion of the prodrug into the toxin only occurs in the target tissue, i.e. tumor. Such promoters are e.g. IPTG-, antibiotic-, heat-, pH-, light-, metall-, aerobic-, host cell-, drug-, cell cycle- or tissue specific-inducible promoters.

For example, the enzyme can be glucuronidase converting the less toxic form of the chemoterapeutic agent glucuronyldoxorubicin into a more toxic form. Preferably, the gene encoding such an enzyme is directed by a promoter which is a constitutive or inducible promoter. Further examples of enzyme/prodrug pairs which are useful in the pharmaceutical composition of the present invention are listed in Table 4, above.

Moreover, the delivery system of the present application even allows the application of compounds which could so far not be used for tumor therapy due to their high toxicity when systemicly applied. Such compounds include proteins inhibiting elongation factors, proteins binding to ribosomal subunits, proteins modifying nucleotides, nucleases, proteases or cytokines (e.g. IL-2, IL-12 etc.), since experimental data suggest that the local release of cytokines might have a positive effect on the immunosuppressive status of the tumor.

Furthermore, the LIVP strain of *Vaccinia* virus can contain a BAC (Bacterial Artificial Chromosome) or MAC (Mammalian Artificial Chromosome) encoding several or all proteins of a specific pathway, e.g. antiangiogenesis, apoptosis, wound-healing or anti-tumor growth. Additionally the cell can be a computer-designed cyber virus endocing these proteins in multiple combinations.

For administration, the LIVP strain of *Vaccinia* virus used in the present invention is preferably combined with suitable pharmaceutical carriers. Examples of suitable pharmaceutical carriers are well known in the art and include phosphate buffered saline solutions, water, emulsions, such as oil/water emuslions, various types of wetting agents, sterile solutions etc.. Such carriers can be formulated by conventional methods and can be administered to the subject at a suitable dose. Administration of the virus may be effected by different ways, e.g. by intravenous, intraperitoneal, subcutaneous, intramuscular, topical or intradermal administration. The preferred route of administration is intravenous injection. The route of administration, of course, depends on the nature of the tumor and the kind of virus contained in the pharmaceutical composition. The dosage regimen will be determined by the attending physician based on various clinical factors. As is well known in the medical arts, dosages for any one patient depends on many factors, including the patient's size, body surface area, age, sex, the particular compound to be administered, time and route of administration, the kind, size and localization of the tumor, general health and other drugs being adminstered concurrently.

Preferred tumors that can be treated with the LIVP strain of *Vaccinia* virus of the present invention are bladder tumors, breast tumors, prostate tumors, brain tumors, colon tumors, lung tumors, ovarial carcinomas, and pancreatic carcinomas; liver tumors, skin tumors.

### Brief description of the drawings

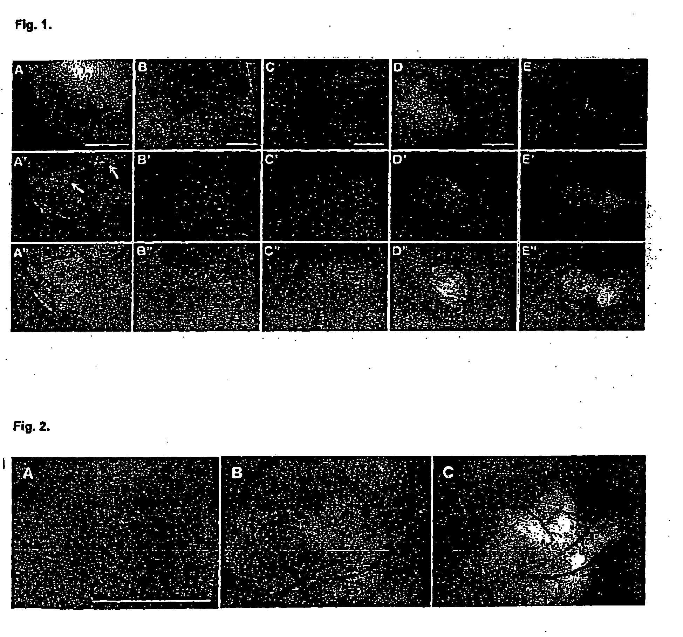

Figure 1: External imaging of GFP expression in subcutaneous C6 glioma tumors in nude mice
   C6 glioma cells (5 x 10⁵) were implanted subcutaneously into the right lateral thigh. At designated days after tumor cell implantation, the animals were infected intravenously with 1 x 10⁸ pfu of rVV*-ruc-gfp* virus particles. GFP expression was monitored under a fluorescence stereomicroscope. Bright field (top), fluorescence (middle), and bright field, fluorescence overlay (bottom) images of subcutaneous glioma tumor are shown. GFP signal can be observed in tumors as small as 22 mm³ in size (B-B"), or as old as 18 days (about 2500 mm³ in size) (A-A"). In older tumors, GFP expression was seen in "patch"-like patterns (indicated by arrows in A'). Marker gene expression in the tumor of the same animal can be monitored continuously 4 (C-C"), 7 (D-D"), and 14 (E-E") days after intravenous viral injection. (Bars = 5 mm.)
Figure 2: Visualization of tumor angiogenesis
   C6 glioma cells (5 x 10⁵) were implanted subcutaneously into the right lateral thigh of nude mice. Ten days after tumor cell implantation, the animals were infected intravenously with 1 x 10⁸ pfu of rVV*-ruc-gfp.* GFP expression was monitored 7 days post-viral injection. Vascularization at the surface of the subcutaneous C6 glioma tumor is shown against the bright green fluorescent background in the tumor following Vaccinia-mediated gene expressions. Bright field (A), fluorescence (B), and bright field, fluorescence overlay (C) images of subcutaneous glioma tumor are illustrated. (Bars = 5 mm.)

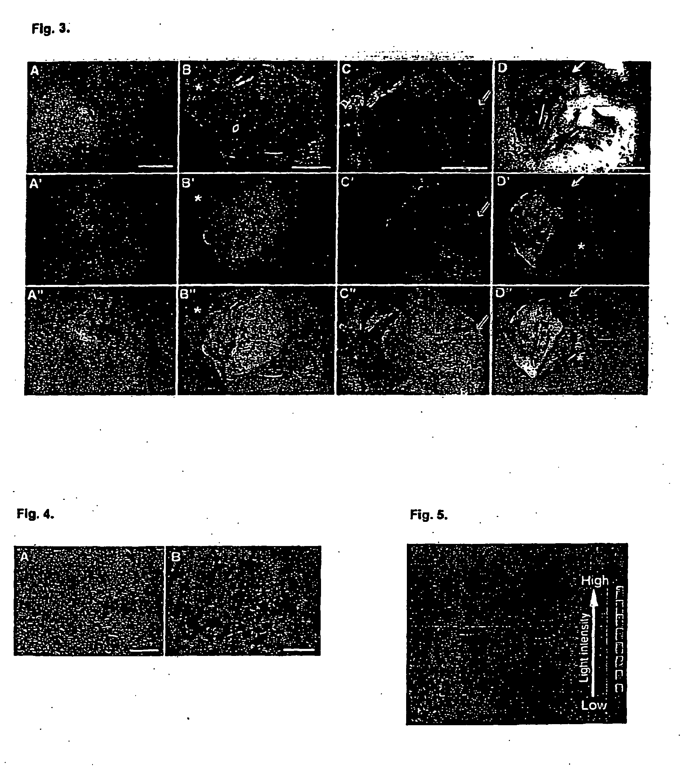

Figure 3: Expression of GFP in subcutaneous glioma tumor of the same animal
   Five days after the subcutaneous implantation of 5 x 10⁵ C6 glioma cells into the right lateral thigh, 10⁸ of rVV*-ruc-gfp* virus particles were injected intravenously. Five days after viral injection, the animal was anesthetized and sacrificed for analysis of GFP expression under fluorescence microscope. The tumor was visualized externally (A-K), with the overlying skin reflected (B-B"), in cross section (C-C"), and in the amputated leg (D-D''). Bright field (A), fluorescence (B), and bright field, fluorescence overlay (C) images of subcutaneous glioma tumor are illustrated. The strongest GFP expressions are seen as patches located along the outer surface of the tumor on the right (double arrows in C-C"). Sharp difference of GFP expression in tumor tissue and in the normal muscle tissue (arrows in D-D") is clearly visible. Asterisks mark the reflected skin (B-B" and D-D''). (Bars = 5 mm.)
Figure 4: Bright field (A) and fluorescence (B) images of tumor cells expressing GFP
   Frozen sections (30 µm thick) of the glioma tumor tissues were prepared from a nude mouse that has been intravenously injected with 1 x 10⁸ of rVV-*ruc-gfp* virus particles. (Bars = 50 µm.)
Figure 5: Low light image of the anesthetized nude mouse to indicate the location of *Renilla* luciferase-triggered light emission in the presence of intravenously injected substrate coelenterazine (5 µg ethanol solution)
Figure 6: Monitoring tumor-specific viral infection based on GFP gene expression in a variety of tumor models
   including subcutaneous PC-3 human prostate tumor (A-A") and MCF-7 human breast tumor (B-B") in nude mice, intracranial C6 rat glioma tumor (C-C", arrows indicate the location of the tumor) in Lewis rats, and MB-49 mouse bladder tumor (D-D") in C57 mice. Animals were monitored 7 days after intravenous injections of 1 x 10⁸ of rVV-*ruc*-*gfp* virus particles. Bright field (top), fluorescence (middle), and bright field, fluorescence overlay (bottom) images of the tumor are illustrated. (Bars = 5 mm.)

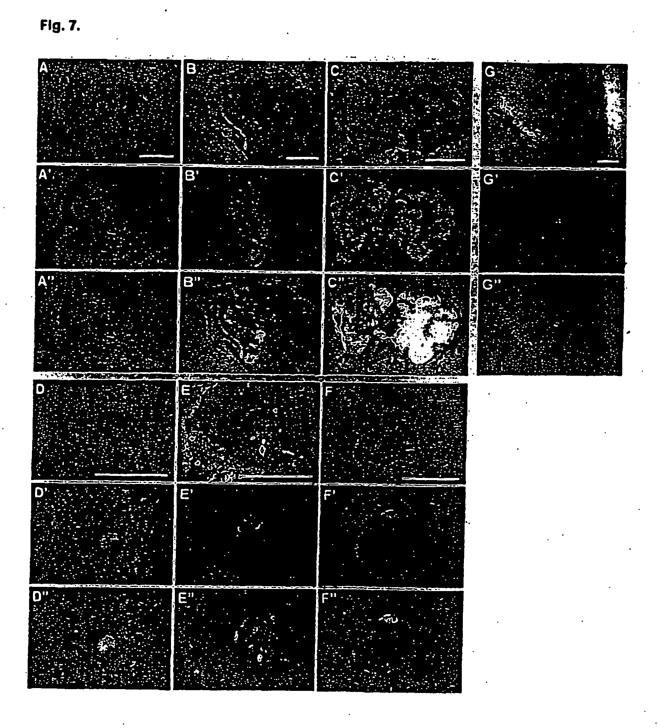

Figure 7 : Monitoring Vaccinia-mediated GFP expression in a breast tumor model
   Nude mouse carrying breast tumor was injected intravenously with 1 x 10⁸ of rVV-*ruc-gfp* virus particles. Both the primary tumor (A-A", B-B", and C-C") and the metastasized tumor (D-D", E-E", and F-F") were visualized externally (A-A" and D-D"), with overlying skin removed (B-B" and E-E"), and when they were split open (C-C" and F-F") in a set of bright field, fluorescence (`) and bright field, fluorescence overlay (`') images. GFP expression in lung metastases in the same animal was also visualized (G-G"). (Bars = 5 mm (A-A" to F-F"), and Bars = 1 mm (G-G'').

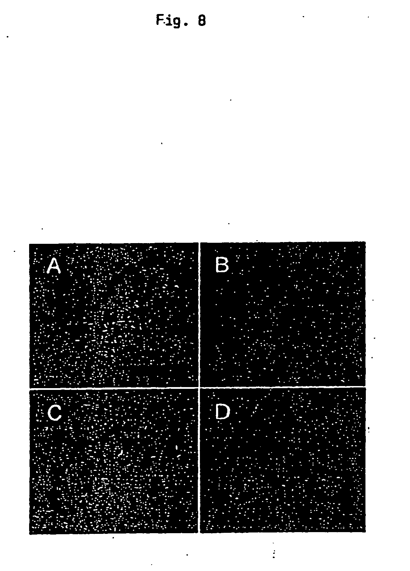

Figure 8: Visualization of the clearance of light emitting bacteria from nude mice based on the detection of light emission under the low light imager
   Nude mice were intravenously injected with 10⁷ cells of attenuated *S. typhimurium* (A, B) and *V. cholera* (C, D). Both strains were transformed with pLITE201 carrying the *lux* operon. Photon collection was done 20 min (A, C) and 2 days (B, D) after bacterial injections.

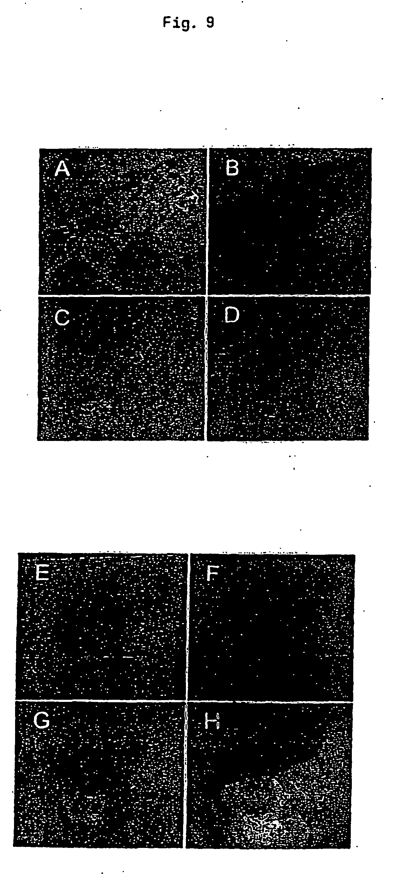

Figure 9: Homing of glioma tumors by attenuated bacteria
   Nude mice with a C6 glioma tumor in the right hind leg were intravenously injected with 10⁷ attenuated *S. typhimurium* (A-D) and with *V. cholera* (E-H) both transformed with pLITE201 plasmid DNA encoding the *lux* operon. Photon collection was carried out for one minute under the low light imager. Mice injected with S. *typhimurium* exhibited luminescence immediately through the whole animal (A). In contrast, luminescence in the mice injected with *V. cholera* was visible in the liver area (E). Two days after bacterial injection, both groups of mice demonstrated luminescence only in the tumor region (B, F). The light emission in the tumors infected with *S. typhimurium* slowly diminished four (C) and six (D) days after bacterial injection. Tumors infected with *V. cholera* showed enormously increased light emission four (G) and six (H) days after injection suggesting continued replication of the bacteria in the tumor tissues.

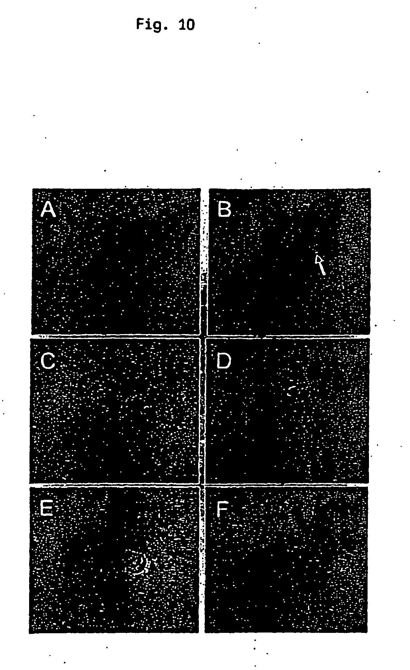

Figure 10: Homing in of bacteria to breast tumors
   Nude mice with breast tumors in the right breast pad were intravenously injected with 10⁷ attenuated *V. cholera* (A-D) or with 10⁷ *E. coli* (E-F) transformed with pLITE201 plasmid DNA encoding the *lux* operon. Photon collection was carried out for one minute under the low light imager. Twenty minutes after bacterial delivery, luminescent *V. cholera* were observed in the liver (A). Forty-eight hours after injection, light emission was noted in the primary breast tumor in the right breast area and a metastatic tumor (arrow) in the left breast area, and in the incision wound (B). At five days, the light emission was visible only in the tumor regions, and not at the wound (C). Eight days after bacterial injection, the luminescent activity was abolished from the smaller tumor region but remained strong in the primary breast tumor (D). Homing in of *E. coli* to breast tumors in nude mice was also observed two days after intravenous bacterial injection (E: side view, F: ventral view).

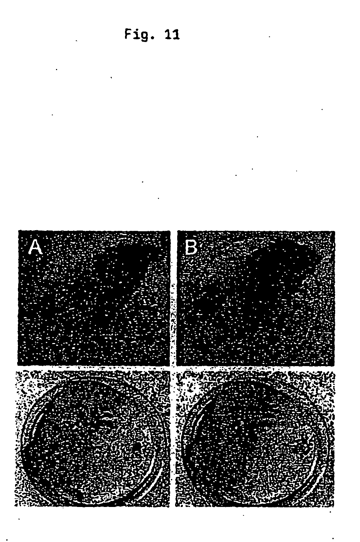

Figure 11: Homing in of bacteria to bladder tumors in C57 mice
   C57 mice were intravenously injected with 10⁷ attenuated *V. cholera* transformed with pLITE201 encoding the *lux* operon. Nine days after bacterial delivery, luminescence was noted in the bladder region of the whole animal (A). The animal was sacrificed and an abdominal incision was made to expose the bladder. The light emission was limited to the bladder region (B). With the removal of the bladder (C) from the mouse, the entire source of light emission was removed (D) as demonstrated by the overlay of the low light photon emission image over the photographic image of the excised bladder.

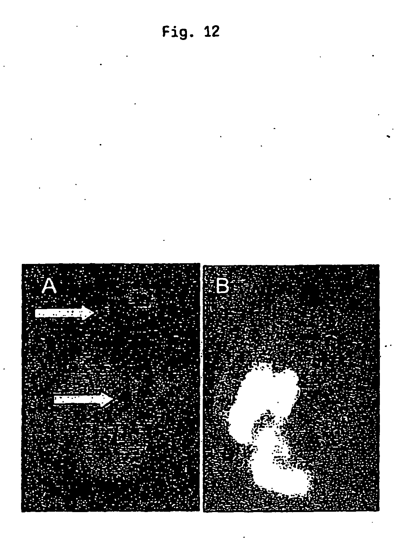

Figure 12: Homing in of bacteria to brain glioma tumors in Lewis rats
   Lewis rats were intravenously injected with 10⁸ cells of attenuated *V. cholera* transformed with pLITE201 encoding the *lux* operon. Twenty-four hours after bacterial injection, faint luminescence was noted in the head region of the whole animal during visualization under the low light imager. The animals were sacrificed and their brain removed. Photon collection was carried out for one minute from rats with (A) and without (B) brain tumors. Strong luminescence was confirmed in regions of the brain of the rats with the brain tumor (marked with arrows in A). Luminescence was completely absent in the control brain tissues (B).

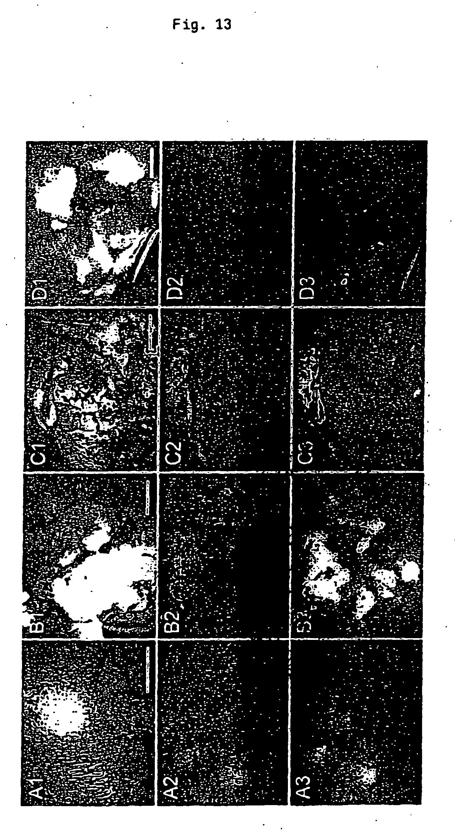

Figure 13 : Transformed human fibrosarcoma cells home in on subcutaneous glioma tumors in nude mice
   Nude mice with subcutaneous glioma tumors were injected intravenously with 5 x 10⁵ human fibrosarcoma cells, which were permanently transformed with retrovirus derived from pLEIN. Seven days post-injection, the animals were anesthetized and monitored under a fluorescent stereomicroscope. Fluorescent cells were noted only in the tumor region of the whole mice through the skin (A1-3). Upon exposure of the tumor tissues by reflection of the overlying skin (B1 -3), and in cross sections of the tumors (Cl -3), fluorescent patches were visible in distinct regions. Close examination of the organs of the mice showed the presence of small clusters of fluorescent cells in the lungs of the animals, demonstrating the affinity of the fibrosarcoma cells for the lungs in addition to the tumorous tissues (D1-3). (Bars = 5 mm (A1-C3), = 1 mm (D1-D3)).

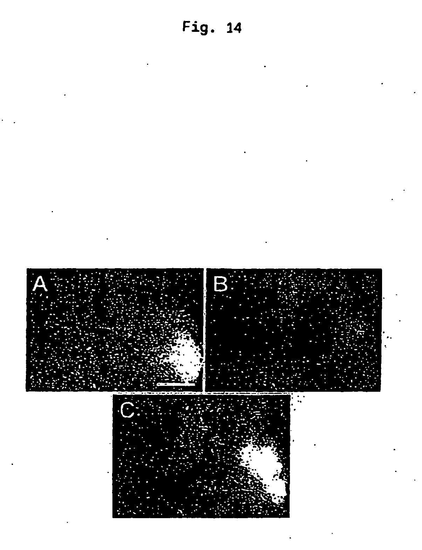

Figure 14: Homing of attenuated *Listeria monocytogenes* into subcutaneous prostate tumors
   Nude mice with subcutaneous human PC3 prostate tumor in the right hind leg were intravenously injected with 10⁷ attenuated *L. monocytogenes* transformed with psod-*gfp* plasmid DNA carrying the *gfp* cDNA, GFP fluorescence was observed under a fluorescence stereo microscope. Twenty-seven hours after bacterial injection, GFP signal was detected only in the tumor region. The tumor is shown in a set of visible light (a), fluorescent (b), and visible and fluorescent light overlay (C) images. (Bars = 5 mm.)

The present invention is explained by the examples 1 and 2. Examples 3 to 7 are comparative.

### Example 1: Materials and Methods

**(A) Bacterium strains.** The bacterial strains used were attenuated *Salmonella typhimurium* (SL7207 hisG46, DEL407[aroA544::Tn10]), attenuated *Vibrio cholerae* (Bengal 2 Serotyp 0139, M010 DattRS1), and attenuated *Listeria monocytogenes* (D2 mpl, actA, plcB).The bacterial strains were kindly provided by Prof. W. Göbel (University of Würzburg, Germany).
**(B) Plasmid constructs.** The plasmid pLITE201 containing the *luxCDABE* cassette was obtained from Dr. Marines, Biotech 24,1998, 56-58). The plasmid pXylA-dual with the operon sequence of *gfp*-cDNA, *lux* AB, lux *CD,* and *lux* E under the control of the Xylose promoter was kindly provided by Dr. Phil Hill (University of Nottingham, UK).
**(C) Transformation of bacteria**
   The bacteria were transformed by electroporation.
**(D) Tumor Cell lines.** The rat C6 nitrosourea-induced glioma cell line (ATCC, Rockille, MID) was cultured in RPMI-1640 medium (Cellgro, Mediatech, Inc., Herndon, VA) supplemented with 10% (v/v) FBS and 1 x penicillin/streptomycin. The human PC3 prostate carcinoma cell line (ATCC, Rockville, MD) and the MB-49 mouse bladder tumor cells and rat 9L glioma cells were maintained in DMEM medium (Cellgro, Mediatech, Inc., Herndon, VA) supplemented with L-glutamine and 10% (v/v) FBS. HT1080 fibrosarcoma cells (ATCC, Manassas, VA) were cultured in F12 minimal essential media (Cellgro, Mediatech, Inc., Herndon, VA) supplemented with 10% FBS and 1 x penicillin/streptomycin. The MCF-7 human mammary carcinoma cell line (ATCC, Rockville, MD), permanently transformed with a plasmid carrying pro-IGF-II cDNA (obtained from Dr. Daisy De Leon, Loma Linda University, Loma Linda, CA) was cultured in DMEM/F12 medium supplemented with 5% FBS and 560 µg/ml of G418 (Life Technologies, Grand Island, NY).
**(E) Production and propagation of retrovirus to generate a light-emitting stably transformed cell line.** PT67 packing cells (Clontech, Palo Alto, CA) were cultured in DMEM medium supplemented with 10% (v/v) FBS. At 70% confluence, PT67 cells were transformed with pLEIN (Clontech, Palo Alto, CA) using calcium phosphate precipitation method (Profection Mammalian Transfection Systems, Promega, Madison, WI) for 12 hours. Fresh medium was replenished at this time. Retroviral supernatant collected from PT67 cells 48 hours post transformation was filtered through a 0.45 µm filter and was added to target HT1080 cells along with polybrene to a final concentration of 4 µg/ml. The medium was replaced after 24 hours and the cells were treated with G918 selection at 400 µg/ml and stepwise increased to 1200 µg/ml.
**(F) Propagation of recombinant *Vaccinia* virus.** *Vaccinia* virus Lister strain (LIVP) was used as a wild type virus. Recombinant *Vaccinia* virus rVV-*ruc-gfp* was constructed by inserting, via homologous recombination, the *ruc-gfp*-cassette (Wang et al., Proc. Biolumin. Chemilumin. 9, 1996, 419-422) into the *Vaccinia* virus genome. The virus was amplified in CV-1 cells by addition of virus particles at a multiplicity of infection (MOI) of 0. 1 pfu/cell to CV-1 cell monolayers followed by incubation at 37°C for 1 h with brief agitation every 10 min. At this time, the supernatant fluid with virus particles was removed, and the cell monolayers were washed once with serum free medium. Complete growth medium was then added and the cells were incubated at 37°C. rVV-*ruc-gfp* virions propagated in CV-1 cells were purified through a sucrose gradient. A plaque assay was used 72 h after infection to determine the titer of recombinant virus by staining the cells with 50% crystal violet solution in ethanol.
**(G) Generation of mice carrying tumor implants.** Five- to six-week-old male BALB/c athymic nu/nu mice (25-30 g in body weight) and Lewis rats (250-300 g in body weight) were purchased from Harlan (Frederick, MD). C57BL/6J *Min*/*+* mice were obtained from Jackson Laboratories (Bar Harbor, ME), *Min* (multiple intestinal neoplasia) is an autosomal dominant trait involving a nonsense mutation in codon 850 of the murine *Apc* gene, which renders these animals susceptible to spontaneous intestinal adenoma formation.
   To obtain tumors in nude mice, C6 glioma cells were grown, harvested and the cell number was determined by the Trypan Blue exclusion method. Disinfectant was applied to the skin surface, then approximately 5 x 10⁵ cells were suspended in 100 µl of phosphate buffered saline (PBS) and injected subcutaneously into the right lateral thigh of each mouse. Tumor growth was monitored by recording the size of the tumor with a digital caliper. Tumor volume (mm³) was estimated by the formula (L x H x W)/2, where L is the length, W is the width, and H is the height of the tumor in mm.
   Intracerebral glioma tumors were generated by injecting C6 glioma cells into the head of rats. Prior to injection, rats were anesthetized with sodium pentobarbital (Nembutal® Sodium solution, Abbot Laboratories, North Chicago, IL; 60 mg/kg body weight). A midline scalp incision (0.5 -1 cm) was made, the skin was retracted, and a 1 mm burr hole was made in the skull at a location 2 mm to the left and 2.5 mm posterior to the brigma. Tumor cells were pipetted into an insulin syringe, which was fitted with a 29-gauge needle and mounted in a stereotactic holder. The needle was inserted vertically through the burr hole to a depth of 3 mm. After injection into the brain of 5 x 10⁵ C6 cells in a 10 µl volume, the needle was kept in place for 15 sec and then withdrawn. The skin incision was closed with surgical clips. Mice bearing subcutaneous prostate tumors were generated over a period of one month following subcutaneous implantation of 3 x 10⁶ PC3 human prostate cells.
   MB-49 mouse bladder tumor cells were implanted in the C57 mouse bladder to produce animals with bladder tumors. To generate animals with breast cancer (Tian and DeLeon, submitted for publication), female nude mice were first implanted with 0.72 mg/90 day-release 17β-estradiol pellets (Innovative Research, Rockville, MD) in the skin to facilitate breast tumor development and metastasis. One day after estrogen pellet implantation, 1 x 10⁶ MCF-7 human breast carcinoma cells transformed with pro-IGF-II (Dull et al., Nature 310 (1984), 777-781) were implanted in the mammary fat pad. For orthotopic transplants, tumors developed from implanted cells were resected and minced into 1-mm³ cubes for tissue transplantation into the mammary fat pad.
**(H) Assay of *Renilla* luciferase in live animals.** Mice were anesthetized with Nembutal (60 mg/kg body weight) before every *Renilla* luciferase assay. *Renilla* luciferase activities were determined after intravenous injection of a mixture of 5 µl of coelenterazine (0.5 µg/µl diluted ethanol solution) and 95 µl of luciferase assay buffer (0. 5 M NaCl; 1 mM EDTA; and 0. 1 M potassium phosphate, pH 7.4). Whole live animals were then imaged in a dark box using the Hamamatsu ARGUS100 low light video camera, and the images were recorded using Image Pro Plus 3.1 software (Media Cybernetics, Silver Spring, MD). The pseudocolored photon emission image was superimposed onto the gray scale image of the animal in order to precisely locate the site of light emission.
**(I) Detection of luminescence and fluorescence.** Immediately before imaging, mice and rats were anesthetized with Nembutal® (60 mg/kg body weight). The animals were placed inside the dark box for photon counting and recording superimposed images (ARGUS 100, Hamamatsu, Hamamatsu, Japan). Photon collection was for one minute from ventral and dorsal views of the animals. A light image was then recorded and the low light image was then superimposed over the light image to record the location of luminescent activity.
   Imaging of GFP expression in tumors of live animals was performed using a Leica MZ8 stereo fluorescence microscope equipped with a mercury lamp power supply and a GFP filter (excitation at 470 nm). Images were captured using a SONY DKC-5000 3CCD digital photo camera.
**(J) Histology of tumor tissues**. Under anesthesia, the animals were euthanized with an overdose of Nembutal®. The tissues of interest were removed, embedded in Tissue-Tek OCT compound (Miles Scientific, Naperville, IL) and immediately frozen in liquid nitrogen without fixation. Frozen sections were cut at -20°C using a Reichert-Jung Cryocut 1800 cryostat. GFP fluorescence of the tissues was monitored under a Leica fluorescence microscope and the images were recorded using Photoshop software.

### Example 2: Results obtained by intravenous injection of recombinant Vaccinia virus rVV-ruc-gfp into mice

### (A) Monitoring of virus-mediated marker gene expression in immunodeficient mice

*Vaccinia* virus (1 x 10⁸ pfu) carrying the *Renilla* luciferase - GFP fusion expression cassette (rVV*-ruc-gfp*) was introduced intravenously into nude mice with no tumors. The animals were observed once every 3 days over a two-week time period under the low-light imager to monitor luciferase catalyzed light emission immediately after intravenous injection of coelenterazine, and under a fluorescence microscope to visualize GFP expression. Neither apparent luminescence nor green fluorescence was detected in the animals when imaged externally, except at certain locations that had small skin lesions. Such luminescence and fluorescence signals disappeared after a few days once the lesions had healed. Animals were sacrificed one week and two weeks after viral infection, and their organs were removed and examined for the presence of luminescence and GFP fluorescence signals. One week after viral injection, no luminescence or green fluorescence could be detected in brain, liver, lung, spleen, kidney or testis. These results indicated that the rVV*-ruc-gfp* virus did not show organ specificity after injection and that the virus seemed to be cleared from the animal by the immune system soon after systemic delivery via the bloodstream.

### (B) Visualization of Vaccinia virus-mediated marker gene expression in glioma tumors of live nude mice

The distribution of injected *Vaccinia* virus in nude mice bearing subcutaneously implanted C6 glioma tumors was examined. Nude mice with tumors approximately 500 mm³ in size were injected intravenously with 1 x 10⁸ pfu of the rVV-*ruc-gfp* virus. Seven days after virus injection, the animals were monitored for GFP expression under a fluorescence microscope to determine the presence of viral infection and multiplication in the tumors, which had grown to approximately 2500 mm³ in size. Surprisingly, green fluorescence was detected only in the tumor regions in live animals. Seven days after viral injection, the GFP fluorescence was very intensely localized in a patch-like pattern restricted to the tumor region (Fig. 1A-A"). These patches, often seen at the end of blood vessel branches, may have indicated local viral infection of tumor cells that surround the leaky terminals of capillary vessels. During real-time observation of the same tumors, the GFP signal from the center of these patches started to disappear, and new green fluorescent centers appeared in the form of rings at the periphery of the fading patches. The new sites of intense GFP fluorescence may have resulted from progression of the viral infection to nearby cells within the tumor during tumor growth and expansion. After careful examination of the mice, with the exception of the tumor region, no detectable green fluorescence was seen elsewhere on the body surface or in the dissected organs. This experiment clearly showed that a mature solid tumor could be easily localized by the labeled *Vaccinia* virus, based on light-emission, and it also demonstrated the affinity of virus particles for the tumor tissue.

To determine whether tumor size and vascularization are decisive factors for viral retention in tumors, nude mice were intravenously injected with 1 x 10⁸ rVV-*ruc*-*gfp Vaccinia* virus particles one day after subcutaneous C6 cell implantation. Surprisingly, 4 days after viral injection GFP expression was seen in 5-day-old C6 tumors that had a volume of about 25 mm³ (Fig. 1B-B''). Examination of labeled *Vaccinia* virus tumor targeting by visualization of GFP expression in implanted tumors younger than 5 days was not feasible in live mice, since sufficient levels of marker gene expression required approximately 4 days to allow detection under a fluorescence microscope.

The finding that injection of the rVV-*ruc*-*gfp Vaccinia* virus into the bloodstream of the host resulted in GFP expression and accumulation in tumors suitable for non-invasive tumor detection allowed us to follow the entry and replication process of this virus in the same animal in real time (Fig. 1 C-C", D-D" and E-E"). A continuously increasing level of GFP fluorescence was observed in the same animal throughout 20 days following viral injection, which was the time scheduled before sacrificing the animals. Such an increase in detectable fluorescence was indicative of a very strong viral replication in the tumor tissue, the latter appearing to function as a protective immunoprivileged environment for viral replication. The viral replication in the tumors was verified by determining the viral titer and light emission of the isolated viral particles in cell cultures. Interestingly, the location of blood vessels and the neovascularization within the periphery of the enlarging tumor were readily visible and confirmed by external visualization against a bright green fluorescent background (Fig. 1A-A", D-D", E-E" and Fig. 2).

To determine the location of viral infection within the tumors, the animals were sacrificed and the skin over the tumor was carefully reflected to expose the tumor. In the exposed tumor, GFP fluorescence was found to be concentrated exclusively in the tumor tissue (Fig. 3B-B" and D-D"). The non-tumorous thigh muscles did not show any fluorescence of viral infection, as indicated by arrows in Fig. 3D-D". The skin overlying the tumor was also non-fluorescent (indicated by asterisks in Fig. 3B-B" and D-D"). Cross sections of the tumor, however, revealed that strong green fluorescent regions were mostly found as patches in the periphery of the tumor (double arrows in Fig. 3 C-C") where the actively dividing tumor cells are presumably located.

To further examine the pattern of viral infection in C6 glioma tumors based on GFP expression, the tumor tissues were sectioned for microscopic analysis under the fluorescence microscope. Comparative analysis of various tissue sections revealed that GFP fluorescence was present in large clusters of cells within the tumor (Fig. 4), but no fluorescence was visible in normal tissues such as the heart, lung, liver, spleen, and kidney.

In addition to GFP, the recombinant rVV-*ruc-gfp* virus carried a second marker gene, which encoded the Renilla luciferase in the form of a fusion protein with GFP. Therefore we were able to directly superimpose the site of GFP fluorescence with light emission from *Renilla* luciferase in the tumors. Immediately after coelenterazine (substrate for *Renilla* luciferase) was delivered by intravenous injection, a very strong luciferase activity was recorded only in the tumor region under a low light video camera (Fig. 5). By lowering the sensitivity of the low light video camera to avoid saturation of light detection, we were able to identify the *Renilla* luciferase gene expression in localized patches in the periphery of the tumor. These patch-like patterns precisely correlated with the GFP signals.

### (C) Affinity of Vaccinia virus delivered to the bloodstream for different tumors implanted into animals

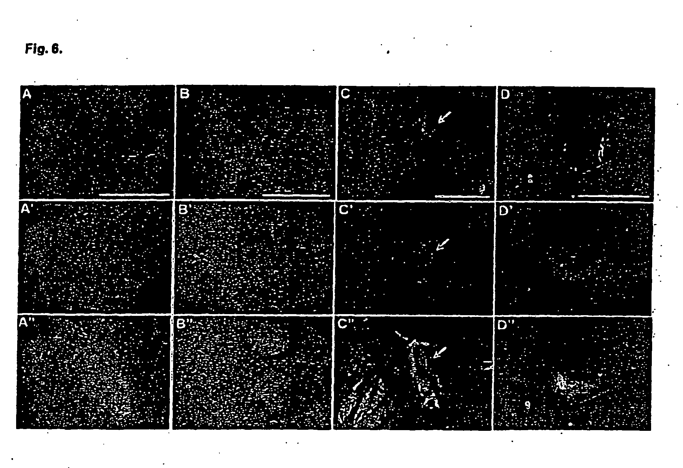

To determine whether the attraction of the *Vaccinia* virus was limited to glioma tumors or whether this attraction could be observed in other tumors, recombinant *Vaccinia* virus was recombinantly introduced into mice that carried different types of implanted tumors. One of these tumor models was a nude mouse with implanted subcutaneous PC-3 human prostate carcinoma. Although the PC3 implants from which tumors developed grew at a much slower rate than the implanted subcutaneous glioma tumors, these tumors showed the same dynamics with regards to *Vaccinia* virus infection when identical titers (1 x 10⁸) were injected intravenously (Fig. 6A-A"). Similar to our findings with glioma tumors, GFP expression was initially detected 4 days after virus injection, and the fluorescence lasted throughout the 3-week observation period.

Female nude mice with established breast tumors were also used for labeled *Vaccinia* injections. These breast tumors were allowed to grow for 6 months after the animals received implants of MCF-7 human breast carcinoma cells transformed with *pro*-*IGF-11* cDNA. At the time of *Vaccinia* virus injection, the tumors had reached maximum growth and the tumor volume (about 400-500 mm³) did not change significantly during the experimental period. Similar to previous experiments, 6 days after intravenous delivery of 1 x 10⁸ rVV-*ruc-gfp* virus particles, strong GFP expression was observed in the breast tumor region (Fig. 6B-B", Fig. 7A-A" and B-B") and nowhere else in the body.

Examination of cross sections of virus-infected breast tumors revealed luminescent "islands" throughout the tumors without any indication of central or peripheral preference of infection (Fig. 7C-C"). The MCF-7 tumor cells used in these breast tumor models are known to metastasize and in addition to the primary solid tumor, a smaller metastasized tumor found on the left side of the body showed GFP fluorescence (Fig. 7 D-D", E-E" , and F-F"). Excised lung tissues were also examined for detection of metastases. Metastasized tumors as small as 0.5 mm in diameter on the surface of the lung were positive for GFP fluorescence (Fig. 7G-G"). The presence of a strong *Renilla* luciferase-mediated light emission confirmed the expression of the luciferase-GFP fusion protein in these breast tumors but nowhere else in the body when the substrate coelenterazine was injected intravenously into the live animals. These experiments showed that intravenously delivered *Vaccinia* virus particles were selectively accumulated to and replicated in primary and metastasized breast tumors in nude mice, likely as a result of the immunocompromised state of the tumor microenvironment.

To determine whether virus particles could move out of tumors and re-enter the circulation, we injected C6 glioma cells into the thigh of mice to form a second tumor in animals already carrying a breast tumor infected with labeled *Vaccinia* virus. If the virus particles were released from the tumor to re-enter the circulation in significant numbers, they would be able to colonize the newly implanted glioma tumor. Monitoring of these second tumors showed that no GFP signal was visible in the new glioma tumor 7 and 14 days after implantation of the glioma cells. To demonstrate that the newly implanted glioma tumors could be targeted by labeled *Vaccinia* virus, a second dose of rVV*-ruc-gfp* virus (1 x 10⁸ pfu) was intravenously injected. Five days later, tumor-specific GFP expression was detected in the newly formed glioma tumor in addition to GFP expression seen in the original breast tumor. These findings suggested that the virus particles in infected tumors were either not released back into the circulation at all, or were not released in sufficient numbers to infect and replicate in a second tumor.

Two additional tumor models, including Lewis rats with intracranial C6 rat glioma tumors and C57 mice with MB-49 mouse bladder tumors in the bladder, were used for *Vaccinia* injections. To determine whether tumor-affinity of virus particles is a phenomenon limited to tumors in nude mice with a diminished T-lymphocyte function or whether it is a general protective property of tumors that may be demonstrated also in immunocompetent animals, Lewis rats with intracranial C6 rat glioma tumors and C57 mice with MB-49 mouse bladder tumors in the bladder were used. A total of 5 x 10⁵ C6 glioma cells in a 100 µl volume were stereotactically implanted in the brains of 2 of 4 immunocompetent Lewis rats, and the tumors were allowed to grow for 5 days. The other 2 rats were injected intracranially with phosphate-buffered saline to serve as controls. On day six, all 4 rats were intravenously injected with rVV-*ruc-gfp* virus particles via the femoral vein. Five days after virus injection, all 4 animals were sacrificed, and their brains were carefully excised for analysis by fluorescence microscopy. GFP expression was detected in the brains with implanted intracranial tumors (Fig. 6C-C") while no GFP expression was seen in the control brains. In parallel experiments, C57 mice, with or without bladder tumors, were divided into two groups. One group was injected intravenously with rVV-*ruc-gfp Vaccinia* virus (1 x 10⁹ pfu) and the other with saline solution as control. Five days after virus injection, the animals were sacrificed and examined under the fluorescence microscope. GFP expression was observed in the bladder tumor region in C 57 mice but not in control mice (Fig. 6D-D'').

Taken together, these experiments show that *Vaccinia* virus particles were selectively accumulated and retained in a variety of tumors, probably protected by the tumor microenvironment, and that they were not able to survive in the non-tumorous tissues of immunocompromised as well as immunocompetent animals. The tumor-targeting process by intravenously injected *Vaccinia* virus carrying the light-emitting dual marker gene demonstrated the ability of the *Vaccinia* virus system to detect primary and metastatic tumors in live animals.

### Comparative Example 3: Results of intravenous injection of bacterial and mammalian light-emitting cells into mice

### (A) Visualization of light emitting bacteria present in whole animals after intravenous injection

To determine the fate of intravenously injected luminescent bacteria in the animals, 10⁷ bacteria carrying the pLITE201 plasmid in 50 µl were injected into the left femoral vein of mice under anesthesia. Following closure of the incision with sutures, the mice were monitored under the low light imager (ARGUS 100 Camera System, Hamamatsu, Hamamatsu, Japan) in real time and photons were collected for one minute. The imaging was repeated in two-day time intervals to determine the presence of light emission from a given animal. It was found that the distribution pattern of light emission following an intravenous injection of bacteria into mice was characteristic of the bacterial strains used. Injection of the attenuated *V. cholera* into the bloodstream resulted in light emission localized in the liver immediately. Injection of *S. typhimurium,* however, was widely disseminated throughout the body of the animal suggesting a difference in the interaction with host cell system (Figure 8A-8D). Imaging the same animals 24 and 48 hours post-infection showed that all of the detectable light emission from the earlier time diminished rapidly and was eliminated completely from the injected animal. These findings suggest that light emitting bacteria injected into the bloodstream via the femoral vein are cleared. This process was confirmed by photon emission analysis of excised organs, which were found to lack light emission. Similar data were obtained in immunocompetent mice and rats suggesting that the removal of bacteria from the blood is efficient in both systems.

### (B) Bacteria home in to glioma tumors in nude mice

To determine if bacteria preferentially colonize tumorous tissues, nude mice with ten-day-old tumors (about 500 mm³) in the right hind leg were injected intravenously via the femoral vein with 10⁷ *S. typhimurium* or 10⁷ *V*. *cholera* in a 50 µl volume of bacterial suspension. Following injection, the incision wounds were sutured and the animals were monitored for six days under the low light imager. At each observation time point, photons were collected for exactly one minute. In mice injected with *S. typhimurium,* luminescent bacteria were disseminated throughout the whole body of the animal similar to the findings in the non-tumorous mice (Figure 9A). Nude mice injected with *V. cholera,* demonstrated luminescent activity only in the liver region during the early observation period (Figure 9E). Regardless of the bacterial strain injected, two days after injection, luminescent activity was observed only in the tumor region (Figures 9B and 9F). Monitoring of the mice under the low light imager on days four and six post-injection showed decreased amounts of detectable luminescence in the tumors of animals injected with *S*. *typhimurium* (Figures 9C and 9D. This finding was in marked contrast with the findings in the tumors of mice injected with *V. cholera,* which demonstrated not only survival but also propagation of the bacteria in the tumor mass with a dramatic increase in light emission (Figures 9G and 9H).

Nude mice bearing subcutaneous human PC3 human prostate tumors in the right hind leg were intravenously injected with 10⁷ attenuated *L. monocytogenes* transformed with psod-*gfp* plasmid DNA carrying the *gfp* cDNA. GFP fluorescence was observed under a fluorescence stereomicroscope. Twenty-seven hours after bacterial injection, GFP signal was detected only in the tumor region (Figure 10). No GFP signal was observed in the rest of the animal.

### (C) Determination of minimum size and age of glioma tumors necessary for bacterial infection.

The purpose of this experiment was to determine whether the size of the tumor has any influence on its ability to be colonized by bacteria. Tumors were induced in the right hind leg of nude mice by subcutaneous injection of glioma cells as described. On days 0, 2, 4, 6, 8, and 10 of tumor induction, attenuated *S. typhimurium* and *V. cholera* with the pLITE201 plasmid were injected intravenously through the femoral vein. Presence of luminescent bacteria in the tumor was determined by photon collection for exactly one minute under the low light imager two and four days post-infection. The tumor volume was also determined by measuring the dimensions with a digital caliper. The earliest time-point when luminescent activity was noted in the tumors was on day eight after tumor induction. Corresponding tumor volumes were approximately 200 mm³.

### (D) Bacteria home in to breast tumors of nude mice

In order to determine whether bacterial colonization of tumors is limited to glioma cells or whether this is a general phenomenon observed with all tumors, female nude mice bearing tumors in the right breast pad were intravenously injected with 10⁷ *V. cholera* in a 50 µl volume of bacterial suspension. The animals were monitored within the first 10 minutes after inoculation under the low light imager for one minute and demonstrated the typical luminescent pattern in the liver region (Figure 11A). Two days later, while the liver had become clear of luminescent bacteria, the breast tumor was colonized by the labeled *V. cholera.* In addition to the main tumor, a metastatic tumor in the left breast demonstrated luminescent activity (Figure 11B). On day five, the animals had cleared the bacteria that colonized the incision wound, however, the tumors remained luminescent (Figure 11C). Figure 11D shows the continued colonization and propagation of the bacteria in the main tumor, while the metastatic, smaller tumor had become cleared. Luminescent activity continued for over 45 days in the right breast tumor. Similar experiments were conducted using *E. coli* to demonstrate that homing in of tumors by bacteria is not strain dependent (Figures 11E and 11F) .

To determine whether the bacteria from the tumor enter the blood circulation in significant quantities to colonize other sites, a second tumor (C6 glioma) was induced in these animals in the right hind leg. The tumor was allowed to grow for 10 days. No luminescent activity was observed in the glioma tumor demonstrating the absence of a significant bacteria that would cause colonization of this tumor. However, when the animal was rechallenged with 10⁷ attenuated *V. cholera* intravenously, the leg tumor showed strong luminescent activity.

The findings of these experiments demonstrate that larger tumors retain bacteria more effectively over time. Furthermore, the bacteria within the tumors do not escape into the blood in sufficient quantities to infect susceptible sites such as other tumors.

### (E) Bacteria home in to bladder tumors in immunocompetent mice

C57 mice were intravenously injected with 10⁷ attenuated *V. cholera* transformed with pLITE201 encoding the *lux* operon. On day nine after bacterial delivery, luminescent activity was recorded by photon collection for one minute under the low light imager. Light emission was noted in the bladder region of the whole animal (Figure 12A). The animals were sacrificed and an abdominal incision was made to expose the bladder. Luminescent activity was positively confirmed to be limited to the bladder (Figure 12B). Upon removal of the bladder from the mice, luminescent activity was no longer visible anywhere in the animals, however, the excised bladders continued to demonstrate light emission (Figure 12C). Based on the results of this experiment, bacteria can target tumors in immunocompetent as well as nude mice. Furthermore, the bacteria can also target smaller tumors.

### (F) Bacteria home in to glioma tumors in the brain of rats

Lewis rats with glioma tumors in the brain were intravenously injected with 10⁸ attenuated *V. cholera* with the pLITE201 plasmid through the left femoral vein to determine if bacteria can cross the blood-brain barrier and target tumors in immunocompetent animals. The whole animals were monitored for one minute under the low light imager the following day and low levels of luminescent activity was observed through the skull. The rats were sacrificed and the brain tissue was removed in one piece in order to further evaluate the exact location of the luminescent bacteria. Visualization of the excised brain under the imager demonstrated strong luminescent activity in specific regions of the brain (Figure 13A). Similar imaging of control rats without brains tumors, which were intravenously injected with the labeled bacteria, demonstrated absence of any luminescent activity (Figure 13B).

### (G) Transformed human fibrosarcoma cells home in to subcutaneous glioma tumors in nude mice

Nude mice with human breast tumors were injected intravenously with 5 x 10⁵ human fibrosarcoma cells, which were permanently transformed with retrovirus derived from pLEIN. Seven days post-injection, the animals were anesthetized with Nembutal, and monitored under a fluorescent stereomicroscope. Fluorescent cells were noted only in the tumor region of the whole mice through the skin (Figure 14A1-3). Upon exposure of the tumor tissues by reflection of the overlying skin (Figure 14B1-3), and in cross sections of the tumors (Figure 14C1 -3), fluorescent patches were visible in distinct regions. Close examination of the organs of the mice showed the presence of small clusters of fluorescent cells in the lungs of the animals, demonstrating the affinity of the fibrosarcoma cells for the lungs in addition to the tumorous tissue.

### Comparative Example 4: Construction of bacterial plasmid vectors that carry the light-emitting protein encoding expression cassettes and the therapeutic gene expression constructs in cis configuration

### (A) Rationale

Using the light-emitting expression systems described above, tumors could be imaged based on light emission for up to 45 days in animals. These findings suggest a remarkable plasmid DNA stability in bacteria in the absence of selection. Therefore, by placing the therapeutic gene cassette in cis configuration with the light-emitting protein expression cassette on the same replicon, light emission can be used as an indicator of therapeutic construct presence and stability. In contrast to light-emitting proteins, the therapeutic proteins, endostatin and *Pseudomonas* exotoxin/TGF alpha fusion protein, are required to be secreted from the bacteria into the medium or into the cytosol of tumor cells for inhibition of tumor growth. To achieve protein secretion from the extracellularly replicating *E. coli* cells into the tumor, two constructs with different signal sequences can be designed. For secretion of endostatin, the ompF signal sequence can be placed upstream of the coding sequence of endostatin, which facilitates the secretion into the periplasmic space. To release the endostatin into the medium, an additional protein, the PAS protein, needs to be coexpressed with endostatin. PAS has been shown to cause membrane leakiness and the release of secreted proteins into the medium (Tokugawa et al., J.Biotechnol. 37 (1994), 33; Tokugawa et al., J.Biotechnol. 35 (1994), 69). The second construct for the secretion of *Pseudomonas* exotoxin/TGF alpha fusion protein from E. *coli* has the OmpA signal sequence upstream of the fusion gene and the release from the periplasmic space into the medium is facilitated by sequences present in domain II of the exotoxin (Chaudhary et al., PNAS 85 (1988), 2939; Kondo et al., J.Biol.Chem. 263 (1988), 9470; Kihara and Pastan, Bioconj.Chem. 5 (1994), 532). To promote secretion of endostatin and *Pseudomonas* exotoxin/TGF alpha fusion protein from *L. monocytogenes*, the signal sequence of listeriolysin (LLO) (Mengaud et al., Infect.Immun. 56 (1988), 766) can be placed upstream of each coding sequence.

For regulation of endostatin and *Pseudomonas* exotoxin/TGF alpha fusion protein expression levels in bacteria, vectors can be generated where the therapeutic protein encoding genes are under the control of the T7 promoter or the P_{spac} synthetic promoter (Freitag and Jacobs, Infect.Immun. 67 (1999), 1844). Without exogenous induction, the levels of the therapeutic proteins are low in *E. coli* and in *L. monocytogenes*. The minimal levels of therapeutic proteins in bacteria provide greater safety following intravenous injection of the engineered bacteria. In the following, the construction of six plasmid DNAs for constitutive and regulated expression of endostatin and *Pseudomonas* exotoxin/TGF alpha fusion protein in *E. coli* and *L. monocytogenes is* described. All plasmids to be transferred into *E. coli* will carry the constitutively expressed bacterial *lux* operon, and all the plasmids to be transferred into *L. monocytogenes* will carry the constitutively expressed *sod-gfp* cassette. Plasmids BSPT#1-ESi and BSPT#2-Pti are able to replicate in *E. coli* only, and plasmids BSPT#3, #4, #5, and #6 replicate in *E. coli* and *L. monocytogenes.*

### (B) Construction of plasmid vectors for protein expression and secretion from E. coli

The construction of the endostatin secretion vector to be used in E. *coli* is as follows. The coding sequence of human endostatin (591 bp) will be amplified by PCR from the plasmid pES3 with the introduction of the required restriction sites on both ends, followed by ligation into a pBluescript (Clontech Corp., USA) cloning vector to generate pBlue-ES. The ompF signal sequence (Nagahari et al., EMBO J. 4 (1985), 3589) is amplified with Taq polymerase and inserted upstream in frame with the endostatin sequence to generate pBlue-omp F/ES. The expression cassette driven by the T7 promoter will be excised, and inserted into the pLITE201 vector described in Example 1(B), above, carrying the lux*CDABE* cassette, to produce the plasmid pLITE-ompF/ES. The sequence encoding the PAS factor (a 76 amino acid polypeptide) will be amplified from the chromosomal DNA of *Vibrio alginolyticus* (formerly named *Achromobacter iophagus*) (NCIB 11038) with Taq polymerase using the primers 5'-GGGAAAGACATGAAACGCTTA3-' and 5'-AAACAACGAGTGAATTAGCGCT-3', and inserted into the multiple cloning sites of pCR-Blunt (Clontech Corp., USA) to create the expression cassette under the control of the lac promoter. The resulting plasmid will be named pCR-PAS. The lac promoter linked to the pas gene will be excised from pCR-PAS and inserted into pLITEompF/ES to yield the final plasmid BSPT#1-ESI.

Plasmid pVC85 (Kondo et al., 1998, J.Biol. Chem. 263: 9470-9475) contains a T7 promoter, followed by an ompA signal sequence, and a sequence encoding domain II and III of *Pseudomonas* exotoxin (PE40). The DNA sequence encoding PE40 will be excised with restriction enzymes and replaced with a fragment of PE37/TGF alpha (*Pseudomonas* exotoxin A 280-613/TGF alpha) obtained from the plasmid CT4 (Kihara & Pastan, 1994, Bioconjug. Chem. 5: 532-538) to create the plasmid pVC85-PE37/TGF alpha. The expression cassette of ompAPE37/TGF alpha linked to the T7 promoter will be excised and inserted into pLITE201 to yield the final plasmid BSPT#2-PTI.

### (C) Construction of plasmid vectors for protein expression and secretion from L. monocytogenes

Genes encoding endostatin or PE37/TGF alpha will be inserted downstream of the listeriolysin (LLO) signal sequence in the plasmid pCHHI to generate pCHHI-ES and pCCHI-PE37/TGF alpha. Constitutive expression of the therapeutic proteins will be obtained by linking the above secretion cassettes to the listeriolysin promoter obtained from the pCHHI vector. The sod-gfp expression cassette, excised from the plasmid *psod-gfp* (Götz et al. PNAS in press.) will be inserted into pCHHI-ES to generate BSPT#3-ESc, and into pCCHI-PE37/TGF alpha to generate BSPT#4-PTc. For the expression of the therapeutic proteins under the control of an IPTG inducible promoter, the listeriolysin promoter in BSPT#3-ESc and BSPT#4-PTc will be replaced with the P_{spac} promoter from the plasmid pSPAC (Yansura and Henner, PNAS USA 81 (1984), 439) to generate BSPT#5-ESi and BSPT#6-PTi. P_{spac} is a hybrid promoter consisting of the *Bacillus subtilis* bacteriophage SPO-1 promoter and the lac operator. IPTG-induced GFP expression from the P_{spac} promoter has been documented in *L. monocytogenes* in the cytosol of mammalian cells.

### Comparative Example 5: Demonstration of the expression of luciferase and GFP in bacteria and verification of the secretion of endostatin and recombinant toxin/TGF alpha fusion protein and their function in cell culture assays

To be able to detect the presence of E. *coli* and *L. monocytogenes* in tumor tissues in live animals, the levels of the constitutively expressed luciferase and GFP in bacteria need to be adequate. Therefore, after transformation of recipient *E. coli* or *L. monocytogenes* with the constructs described in Example 4, the colonies with the highest luciferase light emission or GFP fluorescence will be selected. In addition to characterizing light emission from each selected colony before intravenous injection, the ability of the selected transformants to secret endostatin and *Pseudomonas* exotoxin/TGF alpha fusion protein into the medium needs to be confirmed. The presence of endostatin and *Pseudomonas* exotoxin/TGF alpha fusion protein synthesized within *E. coli* and *L. monocytogenes* will be determined by extracting these proteins from the cell pellet. The secreted proteins in the medium will be concentrated and analyzed by gel separation and the quantity will be determined by Western blotting. It is imperative to determine the percentage of the newly synthesized proteins expressed from each plasmid construct in either *E. coli* or *L. monocytogenes* that is present in the medium. It is also essential to confirm, in addition to constitutive expression of endostatin and *Pseudomonas* exotoxin/TGF alpha fusion protein, that expression can be induced in *E. coli* and in *L. monocytogenes* upon the addition of IPTG to the bacterial culture medium. For the design of future tumor therapy protocols, the relative amounts of protein secreted by the constitutive expression system needs to be compared to the induced expression levels for a defined time period first in bacterial cultures. It is equally essential to determine that both proteins when synthesized in *E. coli* and *L. monocytogenes* are biologically active if generated from the proposed constructs. Both proteins were synthesized previously in *E. coli* and were shown to be active.

The results of the experiments described below should confirm whether endostatin is successfully secreted from *E. coli* using the OmpF signal peptide in combination with PAS pore forming protein expression. These experiments will also show if the PE40/TGF alpha and PE37/TGF alpha fusion proteins are secreted from bacteria using the OmpA signal peptide in combination with domain II of PE. Further, the listeriolysin signal peptide may also facilitate the secretion of endostatin and the chimeric toxin/TGF alpha fusion protein into the medium as well as into the cytosol of infected tumor cells. Using the endothelial cell migration inhibition assay and the protein synthesis inhibition assay, it can be expected to determine that both proteins secreted into the medium are biologically active. The presence and quantities of these proteins may be regulated by replacing the constitutive promoters with promoters that can be induced by IPTG.

In addition to the secretion system described below, alternative secretion systems such as the *E. coli* HlyBD-dependent secretion pathway (Schlor et al., Mol.Gen.Genet. 256 (1997), 306), may be useful. Alternative secretion signals from other gram positive bacteria, such as the *Bacillus sp.* endoxylanase signal peptide (Choi et al., Appl.Microbiol.Biotechnol. 53 (2000), 640; Jeong and Lee, Biotechnol.Bioeng. 67 (2000), 398) can be introduced.

### (A) Confirmation of endostatin and Pseudomonas exotoxin/TGF alpha fusion protein secretion from bacteria into growth medium

*E. coli* strains (DH5α and BL21 (λDE3) will be transformed with BSPT#1-ESi and BSPT#2-PTi plasmid DNA. *L. monocytogenes* strain EGDA2 will be transformed with plasmids BSPT#3-ESc, BSPT#4-PTc, BSPT#5-ESi, and BSPT#6-PTi individually. After plating on appropriate antibiotic-containing plates, individual colonies will be selected from each transformation mixture. These colonies will be screened under a low light imager and fluorescence microscope for luciferase and GFP expression, respectively. Three colonies with the most intense light emission from each transformation batch will be chosen for further studies. To verify the secretion of endostatin and *Pseudomas* exotoxin/TGF alpha fusion protein from each selected transformant, the cells will be grown in minimal medium to log phase. After centrifuging down the bacteria, the supernatants will be passed through a 0.45-µm-pore-size filter, and the bacterium-free medium will be used for precipitation of the secreted proteins. The precipitates will be collected by centrifugation. Pellets will be washed, dried, and re-suspended in sample buffer for protein gel separation. Proteins from aliquots corresponding to 10 µl of bacterial culture will be compared to proteins from 200 µl of culture supernatant after separation in a 10% SDS-polyacrylamide gel. Western blot analysis will be performed using polyclonal antibody against endostatin (following the antibody production protocol described by Timpl, Methods Enzymol. 82 (1982), 472) and monoclonal antibody against TGF alpha (Oncogene Research Products, Cambridge, MA, USA). The optimal growth conditions will be established for secretion by sampling the growth medium at different times during growth. A similar method has been used previously to analyze secreted proteins in *Salmonella typhimurium* culture supernatant (Kaniga et al., J.Bacteriol. 177 (1995), 3965). By use of these methods, the amount of secreted proteins in the bacterial culture medium generated by each of the constructs without induction will be established. To estimate the increase in the amount of secreted proteins in the medium, IPTG-dependent promoter activation experiments will be carried out by adding IPTG to the bacterial culture in log phase for 3 to 6 hours, and the secreted proteins will be assayed as above.

### (B) Verification of the biological activity of endostatin secreted by E. coli and L. monocytogenes using a migration inhibition assay

It has been shown that endostatin inhibits vascular endothelial growth factor (VEGF)-induced human umbilical vein endothelial cell (HUVEC) migration. Thus, the biological activity of endostatin secreted by bacteria can be tested using the HUVEC migration assay provided by Cascade Biologics, Portland, OR. The inhibition of cell migration will be assessed in 48-well chemotaxis chambers (Neuro Probe, Galthersburgs, MD) (Polverine et al., Methods Enzymol. 198 (1991), 440). Bacterium-free supernatant from each secretion construct will be added to HUVECs for preincubation for 30 min. After incubation, the HWECs will be placed in the upper chamber. The migration of HUVECs into the lower chamber induced by VEGF₁₆₅ (R&D Systems, Minneapolis, NIN) will be quantified by microscopic analysis. The concentration of functional endostatin in the medium will be directly proportional to the degree of inhibition of HUVEC migration.

### (C) Testing the cytotoxic activity of secreted recombinant PE toxin in tumor cell cultures

The inhibitory activity of the chimeric toxin in mammalian cells will be measured based on inhibition of *de novo* protein synthesis by inactivating EF-2 (Carroll and Collier, J.Biol.Chem. 262 (1987), 8707). Aliquots of bacterium-free supernatants obtained from the expression of various recombinant PE secretion constructs in *E. coli* and in *L*.̅ *monocytogenes* will be added to the C6 glioma cells or to HCTI 16 colon carcinoma cells. Following treatment with medium, the mammalian cells will be pulsed with [³H]-leucine, and the incorporation will be determined in the protein fraction. To determine the presence of secreted chimeric toxin proteins in *L. monocytogenes*-infected mammalian cells, the bacteria will be eliminated from the medium by gentamicin treatment. The mammalian cells containing *L. monocytogenes* in the cytosol will be lysed, and the released bacteria removed from the lysate by filtration. The mammalian cell lysate containing the secreted chimeric toxins will be assayed in protein synthesis inhibition experiments. The inhibition of [³H]-leucine incorporation in tumor cell culture will be directly proportional to the amount of the biologically active chimeric toxin protein in the medium and cell lysate.

### Comparative Example 6: Determination of the entry, localization and distribution of intravenously injected bacteria in tumors of live animals

### (A) Rationale

Since only a small number of intravenously injected bacteria escape the immune system by entering the tumor, their immediate localization is not possible due to limited light emission in live animals. Their location can only be verified by sectioning the tumor to identify the early centers of light emission. Looking at sections at a later time point, bacteria can be seen throughout the entire tumor due to rapid replication. To determine whether one or multiple bacteria enter through the same site, red fluorescent protein can be used to label the extracellularly replicating E. *coli* and green fluorescent protein for the intracellularly replicating *L. monocytogenes.* By visualizing the distribution of the red and green fluorescence in tissue sections, the entry sites as well as replication and localization of *E. coli* and *L. monocytogenes* can be determined individually and simultaneously in the central or peripheral regions of the tumor. It can be expected that the patterns of entry and distribution obtained in implanted tumors mimic those of spontaneous tumors, accordingly, the bacterium-based diagnosis and protein therapy will become a valid approach.

With the experiments described in section (B), below, the entry, replication, and distribution of light-emitting bacteria in spontaneous tumors can be compared to the distribution patterns in implanted tumors. Further, double-labeling experiments will allow the operator to precisely locate the extracellularly replicating E. *coli* and the intracellularly replicating *L. monocytogenes* in the same tumor sections. Lastly, it can be determined (subsequent to a five-day bacterial colonization) whether bacteria are distributed evenly in the tumors or preferential localization occurs in the periphery of the tumor or in the necrotic center. A possible reduction in bacterial entry into spontaneously occurring tumors due to the immunocompetence of these animals can be overcome by increasing the number of intravenously injected bacteria.

### (B) Intravenous injection of E. coli expressing red fluorescent protein and L. monocytogenes expressing green fluorescent protein into nude mice and into rodents with implanted and spontaneous tumors

*E. coli* (DH5α) carrying the DsRed (Matz et al., Nat.Biotech. 17 (1999), 969) expression cassette under the control of a constitutive promoter are used in this experiment. *L*. *monocytogenes* EGD strain derivatives with in-frame deletion in each of the virulence genes were individually labeled with the green fluorescent protein cassette driven by the constitutive SOD promoter.

The localization and intratumoral distribution of bacteria will first be studied in nude mice with implanted C6 glioma or HCT116 colon carcinoma tumors. C6 glioma or HCT116 colon carcinoma cells (5 x 10⁵ in 100 µl) will be subcutaneously injected into the right hind leg of the animals. Twelve days after tumor cell injection, the animals will be anesthetized, and the left femoral vein surgically exposed. Light-emitting bacteria (1 x 10⁶ cells re-suspended in 50 µl of saline) will be intravenously injected, and the wound incision will be closed with sutures. Tumors will be measured three times a week using a caliper. Tumor volume will be calculated as follows: small diameter x large diameter x height / 2.

The localization of bacteria in the tumor, based on GFP or RFP, will also be analyzed using cryosectioned tumor tissues. A reliable morphological and histological preservation, and reproducible GFP or RFP detection may be obtained using frozen sections after a slow tissue freezing protocol (Shariatmadari et al., Biotechniques 30 (2001), 1282). Briefly, tumor tissues will be removed from the sacrificed animals to a Petri dish containing PBS and dissected into the desired size. The samples will be mixed for 2 h in 4% paraformaldehyde (PFA) in PBS at room temperature. They will be washed once with PBS, and embedded in Tissue-Tek at room temperature, and then kept in the dark at 4°C for 24 h and slowly frozen at -70°C. Before sectioning, the tissue will be kept at -20°C for 30 min. Then, 10- to 50-µm-thick sections will be cut with a Reichert-Jung Cryocut 1800 cryostat and collected on poly-L-lysine (1%)-treated microscope slides. During sectioning, the material will be kept at room temperature to avoid several freezing and thawing cycles. Finally, the sections will be rinsed in PBS and mounted in PBS and kept in the dark at 4°C.

To monitor the entry of light emitting *E*. coli and *L*. *monocytogenes* from the blood stream into the tumor, 27 nude mice will be injected with C6 tumor cells, and 27 nude mice with HCT116 colon carcinoma cells. Twelve days after tumor development, 9 animals from the C6 group and 9 from the HCT116 group will receive an intravenous injection of *E. coli* with the RFP construct. Another 9 animals from each group will receive an intravenous injection of *L. monocytogenes* transformed with the GFP cDNA construct. The third group of 9 animals from each tumor model will receive both *E. coli* and *L. monocytogenes* (1 x 10⁶ cells of each). Five hours, 25 hours, and 5 days after injection, three animals of each treatment group will be sacrificed, their tumors excised, and processed individually as described in the above cryosectioning protocol. After freezing, each tumor will be cut into two halves. One half of the tumor will be used for preparing thick sections (60-75 µm), which will be analyzed under a fluorescence stereomicroscope to observe the distribution of bacteria in the sections of tumors obtained from each time point of the experiment. The regions of interest will be identified, thin sectioned, prepared, and analyzed with laser scanning cytometry and under the confocal microscope followed by image reconstruction.

In parallel experiments, animals with spontaneous tumors, as listed in Table 6, will be obtained and used in intravenous injection experiments with *E. coli* carrying the bacterial *lux* operon. Two animals of each tumor model will be used, and the luciferase light emission monitored daily under the low light imager. It is expected that the spontaneously occurring tumors can be imaged similarly to the implanted tumors based on bacterial luciferase expression. Two of the spontaneous tumor models, mice with adenocarcinoma of the large intestine and mice with adenocarcinoma of the mammary tissue, will be used for bacterial localization experiments following intravenous injection of *E. coli* expressing RFP and *L. monocytogenes* expressing GFP as described above. It can be expected that these experiments will emphasize the significance of the bacterium-based diagnosis and protein therapy system.

**Table 6**

| **Spontaneous tumor animal models** | | | | |
|---|---|---|---|---|
| **Animal species** | **Strain name** | **Tumor description** | **Source** | **Reference** |
| Mouse | 129/Sv-Madh3^{tmIpar} | Spontaneous adenocarcinoma of large intestine | Jackson Laboratories Bar Harbor, ME | Zhu et al., Cell 94 (1988), 703 |
| Mouse | FVB/N-TgN(UPII-SV40T)29Xrw with metastasis | Spontaneous carcinoma to the liver | Jackson Laboratories of bladder Bar Harbor, ME | Zhang et al., Cancer Res. 59 (1999), 3512 |
| Mouse | FVB-neuN(N#202) | Spontaneous adenocarcinoma of mammary tissue | Jackson Laboratories Bar Harbor, ME | Guy et al., PNAS USA 89 (1992), 10578 |
| Rat | F344/CrCrlBR | Spontaneous carcinoma of pituitary | Charles River Laboratories, | Hosokawa et al., Toxicol.Pat hol. 21 Wilmington, MA (1993), 283 |

### Comparative Example 7: Verification of bacterium-mediated tumor targeting and bacterium-secreted protein therapy in rodents with implanted or spontaneous tumors

### (A) Rationale

As shown in the previous examples, intravenous injection of light-emitting bacteria results in entry, replication, and accumulation only in the tumor regions in animals. This process can be monitored by imaging of light emission in tumors. Placing the endostatin and chimeric toxin expressing gene cassettes in cis configuration with a light-emitting gene cassette provides an indirect detection system *in vivo* for their temporal and spacial delivery via bacteria.

The endostatin and chimeric toxin gene cassettes are linked to signal peptide encoding sequences, which facilitate the secretion of these proteins into the extracellular space in the tumor or into the cytosol of infected tumor cells. Both proteins secreted from bacteria into the extracellular space of the tumor are expected to function similarly to directly injected purified proteins. Both proteins secreted from *L. monocytogenes* into the cytosol of the infected tumor cells will resemble the viral delivery system reported earlier for endostatin. The bacterial systems can be used as a constitutive secretion system or as an exogenously added IPTG-activatable secretion system in the tumor. By regulating the expression levels of the therapeutic proteins in bacteria that colonize the tumor, the secreted amount of proteins inhibiting tumor growth can be determined. Without the addition of IPTG, the inhibitory protein secretion from the intravenously injected bacterla will be kept at minimum while in blood circulation. This will provide added safety to the recipient tumorous animals during delivery of bacteria. Using the BSPT system, the onset and duration of the therapy can be controlled by the addition of IPTG. Upon completion of the treatment, the bacterial delivery system can be eliminated by administration of antibiotics, similar to treating a bacterial infection.

### (B) Determination of the effect of endostatin and Pseudomonas exotoxin/TGF alpha fusion protein secreted by E. coli and L. monocytogenes on tumor growth in animals with implanted tumors

The inhibitory effect of endostatin and the cytotoxicity of the chimeric toxin secreted by *E. coli* and *L. monocytogenes* in tumors will be determined as follows. Thirty-five nude mice bearing 10-day-old C6 tumors will be injected with bacterial constructs as follows: (a) Five mice with *E. coli* engineered to secrete endostatin; (b) Five mice with *E. coli* engineered to secrete chimeric toxin; (c) Five mice with *L. monocytogenes* engineered to secrete endostatin; (d) Five mice with *L. nionocytogeties* engineered to secrete chimeric toxin; (e) Five mice with E. *coli* secreting endostatin and chimeric toxin; (f) control group: five mice injected with *E. coli* expressing bacterial luciferase alone, and five mice with *L. monocytogenes* expressing GFP. At the time of bacteria injection, each tumor volume will be determined. Three days after injection, the replication of bacteria in the tumors will be monitored under a low light imager or under a fluorescence stereomicroscope. The light emission and the tumor volume will be measured daily up to 20 days after bacterial injection. Ten days after injection, one animal from each group will be sacrificed and the levels of the secreted proteins present in the tumor tissue will be analyzed using Western blot analysis. These experiments will result in inhibition of tumor growth in endostatin treated animals or a more dramatic tumor regression in animals treated with chimeric toxin proteins. The tumor growth in control animals is not expected to be affected by the bacteria alone.

In a follow-up experiment, mice with spontaneous adenocarcinoma of mammary tissue (strain FVB-neuN(N#202), Table 6) will be used to study the effect of secreted proteins on tumor growth. An experimental scheme identical to that described for the C6 tumor analysis will be used. At the completion of tumor therapy, the presence of endostatin or chimeric toxin in the tumor tissue will be determined by Western blot analysis. An identical experimental design will be used to assay the effect of IPTG-induction of endostatin and chimeric toxin production in bacteria in C6 tumors as well as in the spontaneously occurring breast tumor mouse model. It is expected that multiple IPTG induction of protein expression in bacteria might be required for successful tumor therapy.

At any stage of tumor treatment, it may be required to remove the light emitting and therapeutic gene containing bacteria from the animal. To carry out this experiment, mice with 12-day-old C6 tumors will be intravenously injected with E. *coli* expressing the bacterial luciferase. Three days after injection, antibiotic therapy will be initiated by intraperitoneal administration of gentamicin (5 mg/kg body weight) twice daily, or the newly discovered clinafloxacin (CL960) (Nichterlein et al., Zentralbl.Bakteriol. 286 (1997), 401). This treatment will be performed for 5 days, and the effect of antibiotics on the bacteria will be monitored by imaging light emission from the animals daily.

By completing the above experiments, it is expected that endostatin and chimeric toxin proteins secreted into the tumors will cause the inhibition of tumor growth and measurable tumor regression. It is anticipated that tumor regression will be achieved in both groups of rodents with implanted tumors and with spontaneously occurring tumors. Experiments with simultaneous application of secreted endostatin and chimeric toxin proteins in tumor treatment may give the most promising results. The removal of the engineered bacteria from the tumor by administration of antibiotics is an added safety measure of the bacterium-secreted protein therapy (BSPT).

## Claims

1. Use of an LIVP strain of vaccinia virus in the preparation of a composition for detection of a tumor, tumor tissue, cancer or metastasis in a subject, wherein the virus:
(a) replicates in the subject;
(b) is recognized by the immune system of the subject;
(c) is not pathogenic or is attenuated; and
(d) encodes a detectable protein or a protein that induces a detectable signal, wherein the detectable protein:
binds to a contrasting agent, chromophore or a compound or ligand for visualization of tissues; or
comprises a luminescent and/or a fluorescent protein; or is a luciferase and/or proteins providing a substrate for luciferase.

2. The use of claim 1, wherein the composition is for systemic administration to a subject.

3. The use of claim 1 or claim 2, wherein the composition is for intravenous administration to a subject.

4. The use of claim 1, wherein the composition is for intraperitoneal, subcutaneous, intramuscular or intradermal administration.

5. The use of any of claims 1-4, wherein the tumor, tumor tissue, cancer or metastasis is a breast, prostate, bladder, brain, colon, lung, ovarian, pancreatic, liver or skin tumor, tumor tissue, cancer or metastasis.

6. The use of any of claims 1-5, wherein the virus allows for visualization of a tumor, tumor tissue, cancer or metastasis.

7. The use of any of claims 1-6, wherein the virus allows for external visualization of a tumor, tumor tissue, cancer or metastasis.

8. The use of any of claims 1-7, wherein the virus allows for detection of a tumor, tumor tissue, cancer or metastasis through detection of light.

9. The use of any of claims 1-7, wherein the virus allows for detection of a tumor, tumor tissue, cancer or metastasis based on a signal.

10. The use of claim 9, wherein the signal is detectable by magnetic resonance imaging (MRI), single-photon emission computed tomography (SPECT), positron emission tomography (PET), scintigraphy, gamma camera, a β⁺ detector, a y detector, low light imaging or fluorescence imaging.

11. The use of claim 1, wherein the virus encodes a protein that binds a contrasting agent, chromophore or a compound or ligand for visualization of tissues.

12. The use of claim 1, wherein the virus encodes a luminescent and/or a fluorescent protein.

13. The use of claim 1, wherein the virus encodes a luciferase and/or proteins providing a substrate for luciferase.

14. The use of claim 1, wherein the protein is a detectable protein and is a green fluorescent protein or a red fluorescent protein.

15. The use of claim 13, wherein the luciferase is a bacterial luciferase or a firefly luciferase.

16. The use of claim 1, wherein the detectable protein or a protein that induces a detectable signal is a fusion protein comprising a luminescent and a fluorescent protein.

17. The use of claim 16, wherein the fusion protein comprises a Renilla luciferase and a green fluorescent protein.

18. The use of claim 1, wherein the detectable protein or protein that induces a detectable signal a metal-binding protein.

19. The use of claim 1, wherein the detectable protein or protein that induces a detectable signal is a cell receptor that binds to a detectable ligand for detection of a tumor, tumor tissue, cancer or metastasis in a subject.

20. The use of claim 19, wherein the cell receptor is a transferrin receptor.

21. The use of claim 19 or claim 20, wherein the composition further comprises a detectable ligand to which the receptor binds.

22. The use of claim 21, wherein the ligand is a radionuclide-labeled ligand, a metal-labeled ligand or is a ligand that binds to a metal.

23. The use of any of claims 1-10, wherein the composition further comprises an antimicrobial compound fused to a detectable protein or a protein that induces a detectable signal.

24. The use of any of claims 1-10, wherein the composition further comprises a radiolabeled antimicrobial compound, a metal-labeled antimicrobial compound or an antimicrobial compound labeled with a fluorescent probe.

25. The use of claim 23 or claim 24, wherein the antimicrobial compound comprises lactoferrin.

26. The use of claim 23, wherein the antimicrobial compound is fused to a metal-binding protein.

27. The use of any of claims 1-26, wherein the composition is for detection of a tumor, tumor tissue, cancer or metastasis within the whole body of the live subject.

28. The use of any of claims 1-27, wherein the composition is for detection of a tumor, tumor tissue, cancer or metastasis in the breast of a subject.

29. Use of an LIVP strain of vaccinia virus in the preparation of a composition for systemic administration for treatment of a tumor, tumor tissue, cancer or metastasis in a subject, wherein the virus:
(a) replicates in the subject;
(b) is recognized by the immune system of the subject;
(c) is not pathogenic or is attenuated; and
(d) encodes a detectable protein or a protein that induces a detectable signal, wherein the detectable protein:
binds to a contrasting agent, chromophore or a compound or ligand for visualization of tissues: or
comprises a luminescent and/or a fluorescent protein; or
is a luciferase and/or proteins providing a substrate for luciferase.

30. The use of claim 29, wherein the composition is for intravenous administration to a subject.

31. The use of any of claim 29 or claim 30, wherein the virus comprises DNA encoding a protein for treatment of a tumor.

32. The use of claim 31, wherein the virus encodes a cytotoxic protein, a cytostatic protein, an inhibitor of angiogenesis, a protein that stimulates apoptosis, a protein that inhibits an elongation factor, a protein that binds to a ribosomal subunit, a nucleotide-modifying protein, a nuclease, a protease, a cytokine, an enzyme or a receptor.

33. The use of claim 31, wherein the protein for treatment of a tumor is rsCD40L, Fas-ligand, anti-Fas antibody, TRAIL, TNF, Anti-GA733-2, microcin E492, diphtheria toxin, Pseudomonas exotoxins, shiga toxin, endostatin, glucuronidase, beta-galactosidase, thymidine kinase, horseradish peroxidase, carboxypeptidase G2, cytochrome P450, nitroreductase, cytosine deaminase, uracil phosphoribosyltransferase, carboxylesterase, tyrosinase or verotoxin I.

34. The use of any of claims 29-33, wherein the virus allows for visualization of a tumor, tumor tissue, cancer or metastasis.

35. The use of any of claims 29-34, wherein the virus allows for external visualization of a tumor, tumor tissue, cancer or metastasis.

36. The use of any of claim 29-35, wherein the virus allows for detection of a tumor, tumor tissue, cancer or metastasis through detection of light.

37. The use of any of claims 29-36, wherein the virus allows for detection of a tumor, tumor tissue, cancer or metastasis based on a signal.

38. The use of claim 37, wherein the signal is detectable by magnetic resonance imaging (MRI), single-photon emission computed tomography (SPECT), positron emission tomography (PET), scintigraphy, gamma camera, a β⁺ detector, a γ detector, low light imaging or fluorescence imaging.

39. The use of claim 29, wherein the detectable protein or a protein that induces a detectable signal is a protein that binds la contrasting agent, chromophore or a compound or ligand for visualization of tissues.

40. The use of claim 29, wherein the detectable protein or a protein that induces a detectable signal is a protein that emits lights or induces emission of light.

41. The use of claim 29, wherein the detectable protein or a protein that induces a detectable signal is a luminescent and/or a fluorescent protein.

42. The use of claim 29, wherein the protein that induces a detectable signal is a luciferase.

43. The use of claim 29, wherein the protein that induces a detectable signal is a green fluorescent protein or a red fluorescent protein.

44. The use of claim 42, wherein the luciferase is a bacterial luciferase or a firefly luciferase.

45. The use of claim 29, wherein detectable protein or a protein that induces a detectable signal is a fusion protein comprising a luminescent and a fluorescent protein.

46. The use of claim 45, wherein the fusion protein comprises a Renilla luciferase and a green fluorescent protein.

47. The use of claim 29, wherein DNA encoding a detectable protein or a protein that induces a detectable signal encodes a metal-binding protein.

48. The use of any of claims 29-33, wherein the virus comprises DNA encoding a cell receptor that binds:
(a) a detectable ligand for detection of a tumor, tumor tissue, cancer or metastasis in a subject; and/or
(b) a therapeutic ligand for tumor therapy.

49. The use of claim 48, wherein the cell receptor is a transferrin receptor, EGF receptor or somatostatin receptor.

50. The use of claim 48 or claim 49, wherein the composition further comprises a ligand to which the receptor binds.

51. The use of claim 50, wherein the ligand is a ligand coupled to a toxin, a ligand coupled to a therapeutic protein, a radionuclide-labeled ligand, a metal-labeled ligand or a ligand that binds to a metal.

52. The use of any of claims 29-33, wherein the composition further comprises:
(a) an antimicrobial compound fused to a protein for tumor therapy and/or elimination of metastatic tumors; and/or
(b) an antimicrobial compound fused to a detectable protein or a protein that induces a detectable signal.

53. The use of any of claims 29-33, wherein the composition further comprises a radiolabeled antimicrobial compound, a metal-labeled antimicrobial compound or an antimicrobial compound labeled with a fluorescent probe.

54. The use of claim 51 or claim 52, wherein the antimicrobial compound comprises lactoferrin.

55. The use of claim 51, wherein the antimicrobial compound is fused to a metal-binding protein.

56. The use of any of claims 29-55, wherein the tumor, tumor tissue, cancer or metastasis is a breast, prostate, bladder, brain, colon, lung, ovarian, pancreatic, liver or skin tumor, tumor tissue, cancer or metastasis.

## Patentansprüche

1. Verwendung eines LIVP-Stamms eines Vaccinia-Virus für die Herstellung einer Zusammensetzung für den Nachweis eines Tumors, eines Tumorgewebes, von Krebs oder Metastasen in einem Subjekt, wobei das Virus
(a) in dem Subjekt repliziert;
(b) durch das Immunsystem des Subjekts erkannt wird;
(c) nicht pathogen ist oder abgeschwächt ist; und
(d) ein nachweisbares Protein oder ein Protein, das ein nachweisbares Signal hervorruft, codiert, wobei das nachweisbare Protein:
an ein Kontrastmittel, ein Chromophor oder eine Verbindung oder einen Liganden für das Sichtbarmachen von Geweben bindet; oder
ein lumineszierendes und/oder ein fluoreszierendes Protein umfasst; oder
eine Luciferase und/oder Proteine ist, die ein Substrat für Luciferase liefern.

2. Verwendung nach Anspruch 1, wobei die Zusammensetzung eine Zusammensetzung für die systemische Verabreichung in einem Subjekt ist.

3. Verwendung nach Anspruch 1 oder Anspruch 2, wobei die Zusammensetzung eine Zusammensetzung für die intravenöse Verabreichung in einem Subjekt ist.

4. Verwendung nach Anspruch 1, wobei die Zusammensetzung eine Zusammensetzung für die intraperitoneale, subkutane, intramuskuläre oder intradermale Verabreichung ist.

5. Verwendung nach einem der Ansprüche 1 bis 4, wobei der Tumor, das Tumorgewebe, der Krebs oder die Metastasen ein Tumor, ein Tumorgewebe, ein Krebs oder Metastasen der Brust, der Prostata, der Blase, des Hirns, des Darms, der Lunge, der Eierstöcke, der Bauchspeicheldrüse, der Leber oder der Haut ist/ sind.

6. Verwendung nach einem der Ansprüche 1 bis 5, wobei das Virus das Sichtbarmachen eines Tumors, eines Tumorgewebes, von Krebs oder Metastasen ermöglicht.

7. Verwendung nach einem der Ansprüche 1 bis 6, wobei das Virus das externe Sichtbarmachen eines Tumors, eines Tumorgewebes, von Krebs oder Metastasen ermöglicht.

8. Verwendung nach einem der Ansprüche 1 bis 7, wobei das Virus den Nachweis eines Tumors, eines Tumorgewebes, von Krebs oder Metastasen durch den Nachweis von Licht ermöglicht.

9. Verwendung nach einem der Ansprüche 1 bis 7, wobei das Virus den Nachweis eines Tumors, eines Tumorgewebes, von Krebs oder Metastasen auf der Basis eines Signals ermöglicht.

10. Verwendung nach Anspruch 9, wobei das Signal durch Magnetresonanztomographie (MRI), Einzel-Photonen-Emissions-Computertomographie (SPECT), Positronen-Emissions-Tomographie (PET), Szintigraphie, Gamma-Kamera, einen β⁺-Detektor, einen γ-Detektor, Low Light-Bildgebung oder Fluoreszenz-Bildgebung nachweisbar ist.

11. Verwendung nach Anspruch 1, wobei das Virus ein Protein codiert, das ein Kontrastmittel, ein Chromophor oder eine Verbindung oder einen Liganden für das Sichtbarmachen von Geweben bindet.

12. Verwendung nach Anspruch 1, wobei das Virus ein lumineszierendes und/ oder ein fluoreszierendes Protein codiert.

13. Verwendung nach Anspruch 1, wobei das Virus eine Luciferase und/oder Proteine, die ein Substrat für Luciferase liefern, codiert.

14. Verwendung nach Anspruch 1, wobei das Protein ein nachweisbares Protein ist und ein grün fluoreszierendes Protein oder ein rot fluoreszierendes Protein ist.

15. Verwendung nach Anspruch 13, wobei die Luciferase eine bakterielle Luciferase oder eine Glühwürmchen-Luciferase ist.

16. Verwendung nach Anspruch 1, wobei das nachweisbare Protein oder ein Protein, das ein nachweisbares Signal hervorruft, ein Fusionsprotein ist, das ein lumineszierendes und ein fluoreszierendes Protein umfasst.

17. Verwendung nach Anspruch 16, wobei das Fusionsprotein eine Renilla-Luciferase und ein grün fluoreszierendes Protein umfasst.

18. Verwendung nach Anspruch 1, wobei das nachweisbare Protein oder Protein, das ein nachweisbares Signal hervorruft, ein Metall-bindendes Protein ist.

19. Verwendung nach Anspruch 1, wobei das nachweisbare Protein oder Protein, das ein nachweisbares Signal hervorruft, ein Zellrezeptor ist, der an einen nachweisbaren Liganden für den Nachweis eines Tumors, eines Tumorgewebes, von Krebs oder Metastasen in einem Subjekt bindet.

20. Verwendung nach Anspruch 19, wobei der Zellrezeptor ein Transferrin-Rezeptor ist.

21. Verwendung nach Anspruch 19 oder Anspruch 20, wobei die Zusammensetzung außerdem einen nachweisbaren Liganden umfasst, an den der Rezeptor bindet.

22. Verwendung nach Anspruch 21, wobei der Ligand ein Radionuklid-markierter Ligand, ein Metall-markierter Ligand ist oder ein Ligand ist, der an ein Metall bindet.

23. Verwendung nach einem der Ansprüche 1 bis 10, wobei die Zusammensetzung außerdem eine antimikrobielle Verbindung umfasst, die mit einem nachweisbaren Protein oder einem Protein, das ein nachweisbares Signal hervorruft, verknüpft ist.

24. Verwendung nach einem der Ansprüche 1 bis 10, wobei die Zusammensetzung außerdem eine radiomarkierte antimikrobielle Verbindung, eine Metall-markierte antimikrobielle Verbindung oder eine antimikrobielle Verbindung, die mit einer fluoreszierenden Sonde markiert ist, umfasst.

25. Verwendung nach Anspruch 23 oder Anspruch 24, wobei die antimikrobielle Verbindung Lactoferrin umfasst.

26. Verwendung nach Anspruch 23, wobei die antimikrobielle Verbindung mit einem Metall-bindenden Protein verknüpft ist.

27. Verwendung nach einem der Ansprüche 1 bis 26, wobei die Zusammensetzung eine Zusammensetzung für den Nachweis eines Tumors, eines Tumorgewebes, von Krebs oder Metastasen innerhalb des gesamten Körpers des lebenden Subjekts ist.

28. Verwendung nach einem der Ansprüche 1 bis 27, wobei die Zusammensetzung eine Zusammensetzung für den Nachweis eines Tumors, eines Tumorgewebe, von Krebs oder Metastasen in der Brust eines Subjekts ist.

29. Verwendung eines LIVP-Stamms eines Vaccinia-Virus für die Herstellung einer Zusammensetzung zur systemischen Verabreichung für die Behandlung eines Tumors, eines Tumorgewebes, von Krebs oder Metastasen in einem Subjekt, wobei das Virus
(a) in dem Subjekt repliziert;
(b) durch das Immunsystem des Subjekts erkannt wird;
(c) nicht pathogen ist oder abgeschwächt ist; und (d) ein nachweisbares Protein oder ein Protein, das ein nachweisbares Signal hervorruft, codiert, wobei das nachweisbare Protein:
an ein Kontrastmittel, ein Chromophor oder eine Verbindung oder einen Liganden für das Sichtbarmachen von Geweben bindet; oder ein lumineszierendes und/oder ein fluoreszierendes Protein umfasst; oder
eine Luciferase und/oder Proteine ist, die ein Substrat für Luciferase liefern.

30. Verwendung nach Anspruch 29, wobei die Zusammensetzung eine Zusammensetzung für die intravenöse Verabreichung in einem Subjekt ist.

31. Verwendung nach Anspruch 29 oder Anspruch 30, wobei das Virus DNA umfasst, die ein Protein für die Behandlung eines Tumors codiert.

32. Verwendung nach Anspruch 31, wobei das Virus ein cytotoxisches Protein, ein cytostatisches Protein, einen Inhibitor der Angiogenese, ein Protein, das die Apoptose stimuliert, ein Protein, das einen Elongationsfaktor hemmt, ein Protein, das an eine ribosomale Untereinheit bindet, ein Nucleotidmodifizierendes Protein, eine Nuclease, eine Protease, ein Cytokin, ein Enzym oder einen Rezeptor codiert.

33. Verwendung nach Anspruch 31, wobei es sich bei dem Protein für die Behandlung eines Tumors um rsCD40L, einen Fas-Liganden, einen anti-Fas-Antikörper, TRAIL, TNF, anti-GA733-2, Microcin E492, Diphterietoxin, Pseudomonas-Exotoxine, Shiga-Toxin, Endostatin, Glucuronidase, β-Galactosidase, Thymidinkinase, Meerrettichperoxidase, Carboxypeptidase G2, Cytochrom P450, Nitroreductase, Cytosindeaminase, Uracilphosphoribosyltransferase, Carboxylesterase, Tyrosinase oder Verotoxin I handelt.

34. Verwendung nach einem der Ansprüche 29 bis 33, wobei das Virus das Sichtbarmachen eines Tumors, eines Tumorgewebes, von Krebs oder Metastasen ermöglicht.

35. Verwendung nach einem der Ansprüche 29 bis 34, wobei das Virus das externe Sichtbarmachen eines Tumors, eines Tumorgewebes, von Krebs oder Metastasen ermöglicht.

36. Verwendung nach einem der Ansprüche 29 bis 35, wobei das Virus den Nachweis eines Tumors, eines Tumorgewebes, von Krebs oder Metastasen durch den Nachweis von Licht ermöglicht.

37. Verwendung nach einem der Ansprüche 29 bis 36, wobei das Virus den Nachweis eines Tumors, eines Tumorgewebes, von Krebs oder Metastasen auf der Basis eines Signals ermöglicht.

38. Verwendung nach Anspruch 37, wobei das Signal durch Magnetresonanztomographie (MRI), Einzel-Photonen-Emissions-Computertomographie (SPECT), Positronen-Emissions-Tomographie (PET), Szintigraphie, Gamma-Kamera, einen β⁺-Detektor, einen γ-Detektor, Low Light-Bildgebung öder Fluoreszenz-Bildgebung nachweisbar ist.

39. Verwendung nach Anspruch 29, wobei das nachweisbare Protein oder ein Protein, das ein nachweisbares Signal hervorruft, ein Protein ist, das ein Kontrastmittel, ein Chromophor oder eine Verbindung oder einen Liganden für das Sichtbarmachen von Geweben bindet.

40. Verwendung nach Anspruch 29, wobei das nachweisbare Protein oder ein Protein, das ein nachweisbares Signal hervorruft, ein Protein ist, das Licht emittiert oder die Emission von Licht hervorruft.

41. Verwendung nach Anspruch 29 wobei das nachweisbare Protein oder ein Protein, das ein nachweisbares Signal hervorruft, ein lumineszierendes und/oder ein fluoreszierendes Protein ist.

42. Verwendung nach Anspruch 29, wobei das Protein, das ein nachweisbares Signal hervorruft, eine Luciferase ist.

43. Verwendung nach Anspruch 29, wobei das Protein, das ein nachweisbares Signal hervorruft, ein grün fluoreszierendes Protein oder ein rot fluoreszierendes Protein ist.

44. Verwendung nach Anspruch 42, wobei die Luciferase eine bakterielle Luciferase oder eine Glühwürmchen-Luciferase ist.

45. Verwendung nach Anspruch 29, wobei das nachweisbare Protein oder ein Protein, das ein nachweisbares Signal hervorruft, ein Fusionsprotein ist, das ein lumineszierendes und ein fluoreszierendes Protein umfasst.

46. Verwendung nach Anspruch 45, wobei das Fusionsprotein eine Renilla-Luciferase und ein grün fluoreszierendes Protein umfasst.

47. Verwendung nach Anspruch 29, wobei die DNA, die ein nachweisbares Protein oder ein Protein codiert, das ein nachweisbares Signal hervorruft, ein Metall-bindendes Protein codiert.

48. Verwendung nach einem der Ansprüche 29 bis 33, wobei das Virus DNA umfasst, die einen Zellrezeptor codiert, der
(a) einen nachweisbaren Liganden für den Nachweis eines Tumors, eines Tumorgewebes, von Krebs oder Metastasen in einem Subjekt; und/oder
(b) einen therapeutischen Liganden für die Tumortherapie bindet.

49. Verwendung nach Anspruch 48, wobei der Zellrezeptor ein Transferrin-Rezeptor, EGF-Rezeptor oder Somatostatin-Rezeptor ist.

50. Verwendung nach Anspruch 48 oder Anspruch 49, wobei die Zusammensetzung außerdem einen Liganden umfasst, an den der Rezeptor bindet.

51. Verwendung nach Anspruch 50, wobei der Ligand ein Ligand, der an ein Toxin gekoppelt ist, ein Ligand, der an ein therapeutisches Protein gekoppelt ist, ein Radionuklid-markierter Ligand, ein Metall-markierter Ligand oder ein Ligand, der an ein Metall bindet, ist.

52. Verwendung nach einem der Ansprüche 29 bis 33, wobei die Zusammensetzung außerdem umfasst:
(a) eine antimikrobielle Verbindung, die mit einem Protein für die Tumortherapie und/ oder die Beseitigung metastatischer Tumore verknüpft ist; und/oder
(b) eine antimikrobielle Verbindung, die mit einem nachweisbaren Protein oder einem Protein, das ein nachweisbares Signal hervorruft, verknüpft ist.

53. Verwendung nach einem der Ansprüche 29 bis 33, wobei die Zusammensetzung weiterhin eine radiomarkierte antimikrobielle Verbindung, eine Metall-markierte antimikrobielle Verbindung oder eine antimikrobielle Verbindung, die mit einer fluoreszierenden Sonde markiert ist, umfasst.

54. Verwendung nach Anspruch 51 oder Anspruch 52, wobei die antimikrobielle Verbindung Lactoferrin umfasst.

55. Verwendung nach Anspruch 51, wobei die antimikrobielle Verbindung mit einem Metall-bindenden Protein verknüpft ist.

56. Verwendung nach einem der Ansprüche 29 bis 55, wobei der Tumor, das Tumorgewebe, der Krebs oder die Metastasen ein Tumor, ein Tumorgewebe, ein Krebs oder Metastasen der Brust, der Prostata, der Blase, des Hirns, des Darms, der Lunge, der Eierstöcke, der Bauchspeicheldrüse, der Leber oder der Haut ist/ sind.

## Revendications

1. Utilisation d'une souche de LIVP de virus vaccinal dans la préparation d'une composition pour la détection d'une tumeur, d'un tissu tumoral, d'un cancer ou d'une métastase chez un sujet, dans lequel le virus :
(a) se réplique chez le sujet ;
(b) est reconnu par le système immunitaire du sujet ;
(c) n'est pas pathogène ou bien est atténué ; et
(d) code pour une protéine détectable ou une protéine qui induit un signal détectable, dans laquelle la protéine détectable :
se lie à un agent de contraste, un chromophore ou un composé ou ligand pour la visualisation de tissus ;
ou
comprend une protéine luminescente et/ou fluorescente ; ou est une luciférase et/ou des protéines fournissant un substrat pour la luciférase.

2. Utilisation suivant la revendication 1, dans laquelle la composition est destinée à l'administration systémique à un sujet.

3. Utilisation suivant la revendication 1 ou la revendication 2, dans laquelle la composition est destinée à l'administration intraveineuse à un sujet.

4. Utilisation suivant la revendication 1, dans laquelle la composition est destinée à l'administration intrapéritonéale, sous-cutanée, intramusculaire ou intradermique.

5. Utilisation suivant l'une quelconque des revendications 1 à 4, dans laquelle la tumeur, le tissu tumoral, le cancer ou la métastase est une tumeur, un tissu tumoral, un cancer ou une métastase du sein, de la prostate, de la vessie, du cerveau, du côlon, du poumon, des ovaires, du pancréas, du foie ou de la peau.

6. Utilisation suivant l'une quelconque des revendications 1 à 5, dans laquelle le virus permet la visualisation d'une tumeur, d'un tissu tumoral, d'un cancer ou d'une métastase.

7. Utilisation suivant l'une quelconque des revendications 1 à 6, dans laquelle le virus permet la visualisation externe d'une tumeur, d'un tissu tumoral, d'un cancer ou d'une métastase.

8. Utilisation suivant l'une quelconque des revendications 1 à 7, dans laquelle le virus permet la détection d'une tumeur, d'un tissu tumoral, d'un cancer ou d'une métastase par détection de lumière.

9. Utilisation suivant l'une quelconque des revendications 1 à 7, dans laquelle le virus permet la détection d'une tumeur, d'un tissu tumoral, d'un cancer ou d'une métastase sur la base d'un signal.

10. Utilisation suivant la revendication 9, dans laquelle le signal est détectable par imagerie de résonance magnétique (IRM), tomographie assistée par ordinateur à émission de photon unique (SPECT), tomographie par émission de positron (PET), scintigraphie, utilisation d'une caméra gamma, utilisation d'un détecteur β⁺, utilisation d'un détecteur γ, imagerie à faible lumière ou imagerie à fluorescence.

11. Utilisation suivant la revendication 1, dans laquelle le virus code pour une protéine qui se lie à un agent de contraste, un chromophore ou un composé ou ligand pour la visualisation de tissus.

12. Utilisation suivant la revendication 1, dans laquelle le virus code pour une protéine luminescente et/ou fluorescente.

13. Utilisation suivant la revendication 1, dans laquelle le virus code pour une luciférase et/ou des protéines fournissant un substrat pour la luciférase.

14. Utilisation suivant la revendication 1, dans laquelle la protéine est une protéine détectable et est une protéine fluorescente verte ou une protéine fluorescente rouge.

15. Utilisation suivant la revendication 13, dans laquelle la luciférase est une luciférase bactérienne ou une luciférase de luciole.

16. Utilisation suivant la revendication 1, dans laquelle la protéine détectable ou une protéine qui induit un signal détectable est une protéine de fusion comprenant une protéine luminescente et une protéine fluorescente.

17. Utilisation suivant la revendication 16, dans laquelle la protéine de fusion comprend une luciférase de *Renilla* et une protéine fluorescente verte.

18. Utilisation suivant la revendication 1, dans laquelle la protéine détectable ou la protéine qui induit un signal détectable est une protéine de liaison à un métal.

19. Utilisation suivant la revendication 1, dans laquelle la protéine détectable ou la protéine qui induit un signal détectable est un récepteur cellulaire qui se lie à un ligand détectable pour la détection d'une tumeur, d'un tissu tumoral, d'un cancer ou d'une métastase chez un sujet.

20. Utilisation suivant la revendication 19, dans laquelle le récepteur cellulaire est un récepteur de transferrine.

21. Utilisation suivant la revendication 19 ou la revendication 20, dans laquelle la composition comprend en outre un ligand détectable auquel se lie le récepteur.

22. Utilisation suivant la revendication 21, dans laquelle le ligand est un ligand marqué avec un radionucléide, un ligand marqué avec un métal ou un ligand qui se lie à un métal.

23. Utilisation suivant l'une quelconque des revendications 1 à 10, dans laquelle la composition comprend en outre un composé antimicrobien fusionné à une protéine détectable ou une protéine qui induit un signal détectable.

24. Utilisation suivant l'une quelconque des revendications 1 à 10, dans laquelle la composition comprend en outre un composé antimicrobien radiomarqué, un composé antimicrobien marqué avec un métal ou un composé antimicrobien marqué avec une sonde fluorescente.

25. Utilisation suivant la revendication 23 ou la revendication 24, dans laquelle le composé antimicrobien comprend de la lactoferrine.

26. Utilisation suivant la revendication 23, dans laquelle le composé antimicrobien est fusionné avec une protéine se liant à un métal.

27. Utilisation suivant l'une quelconque des revendications 1 à 26, dans laquelle la composition est destinée à la détection d'une tumeur, d'un tissu tumoral, d'un cancer ou d'une métastase dans la totalité de l'organisme du sujet vivant.

28. Utilisation suivant l'une quelconque des revendications 1 à 27, dans laquelle la composition est destinée à la détection d'une tumeur, d'un tissu tumoral, d'un cancer ou d'une métastase dans le sein d'un sujet.

29. Utilisation d'une souche de LIVP de virus vaccinal dans la préparation d'une composition destinée à l'administration systémique pour le traitement d'une tumeur, d'un tissu tumoral, d'un cancer ou d'une métastase chez un sujet, dans laquelle le virus :
(a) se réplique chez le sujet ;
(b) est reconnu par le système immunitaire du sujet ;
(c) n'est pas pathogène ou bien est atténué ; et
(d) code pour une protéine détectable ou une protéine qui induit un signal détectable ; dans laquelle la protéine détectable :
se lie à un agent de contraste, un chromophore ou un composé ou ligand pour la visualisation de tissus ;
ou
comprend une protéine luminescente et/ou fluorescente ; ou
est une luciférase et/ou des protéines fournissant un substrat pour la luciférase.

30. Utilisation suivant la revendication 29, dans laquelle la composition est destinée à l'administration intraveineuse à un sujet.

31. Utilisation suivant l'une quelconque des revendications 29 et 30, dans laquelle le virus comprend un ADN codant pour une protéine destinée au traitement d'une tumeur.

32. Utilisation suivant la revendication 31, dans laquelle le virus code pour une protéine cytotoxique, une protéine cytostatique, un inhibiteur d'angiogénèse, une protéine qui stimule l'apoptose, une protéine qui inibe un facteur d'élongation, une protéine qui se lie à une sous-unité ribosomale, une protéine de modification de nucléotides, une nucléase, une protéase, une cytokine, une enzyme ou un récepteur.

33. Utilisation suivant la revendication 31, dans laquelle la protéine destinée au traitement d'une tumeur est rsCD40L, un ligand de Fas, un anticorps anti-Fas, TRAIL, TNF, l'Anti-GA733-2, la microcine E492, la toxine diphtérique, des exotoxines de Pseudomonas, la toxine shiga, l'endostatine, la glucuronidase, la bêtagalactosidase, la thymidine-kinase, la peroxydase de raifort, la carboxypeptidase G2, le cytochrome P450, la nitroréductase, la cytosine-désaminase, l'uracil-phosphoribosyltransférase, la carboxy-estérase, la tyrosinase ou la vérotoxine I.

34. Utilisation suivant l'une quelconque des revendications 29 à 33, dans laquelle le virus permet la visualisation d'une tumeur, d'un tissu tumoral, d'un cancer ou d'une métastase.

35. Utilisation suivant l'une quelconque des revendications 29 à 34, dans laquelle le virus permet la visualisation externe d'une tumeur, d'un tissu tumoral, d'un cancer ou d'une métastase.

36. Utilisation suivant l'une quelconque des revendications 29 à 35, dans laquelle le virus permet la détection d'une tumeur, d'un tissu tumoral, d'un cancer ou d'une métastase par détection de lumière.

37. Utilisation suivant l'une quelconque des revendications 29 à 36, dans laquelle le virus permet la détection d'une tumeur, d'un tissu tumoral, d'un cancer ou d'une métastase sur la base d'un signal.

38. Utilisation suivant la revendication 37, dans laquelle le signal est détectable par imagerie de résonance magnétique (IRM), tomographie assistée par ordinateur à émission de photon unique (SPECT), tomographie à émission de positron (PET), scintigraphie, utilisation d'une caméra gamma, utilisation d'un détecteur β⁺, utilisation d'un détecteur γ, imagerie à faible lumière ou imagerie à fluorescence.

39. Utilisation suivant la revendication 29, dans laquelle la protéine détectable ou une protéine qui induit un signal détectable est une protéine qui se lie à un agent de contraste, un chromophore ou un composé ou ligand pour la visualisation de tissus.

40. Utilisation suivant la revendication 29, dans laquelle la protéine détectable ou une protéine qui induit un signal détectable est une protéine qui émet de la lumière ou qui induit une émission de lumière.

41. Utilisation suivant la revendication 29, dans laquelle la protéine détectable ou une protéine qui induit un signal détectable est une protéine luminescente et/ou une protéine fluorescente.

42. Utilisation suivant la revendication 29, dans laquelle la protéine qui induit un signal détectable est une luciférase.

43. Utilisation suivant la revendication 29, dans laquelle la protéine qui induit un signal détectable est une protéine fluorescente verte ou une protéine fluorescente rouge.

44. Utilisation suivant la revendication 42, dans laquelle la luciférase est une luciférase bactérienne ou une luciférase de luciole.

45. Utilisation suivant la revendication 29, dans laquelle la protéine détectable ou une protéine qui induit un signal détectable est une protéine de fusion comprenant une protéine luminescente et une protéine fluorescente.

46. Utilisation suivant la revendication 45, dans laquelle la protéine de fusion comprend une luciférase de *Renilla* et une protéine fluorescente verte.

47. Utilisation suivant la revendication 29, dans laquelle l'ADN codant pour une protéine détectable ou une protéine qui induit un signal détectable code pour une protéine se liant à un métal.

48. Utilisation suivant l'une quelconque des revendications 29 à 33, dans laquelle le virus comprend un ADN codant pour un récepteur cellulaire qui se lie à :
(a) un ligand détectable pour la détection d'une tumeur, d'un tissu tumoral, d'un cancer ou d'une métastase chez un sujet ; et/ou
(b) un ligand thérapeutique pour la thérapie d'une tumeur.

49. Utilisation suivant la revendication 48, dans laquelle le récepteur cellulaire est un récepteur de transferrine, un récepteur de EGF ou un récepteur de somatostatine.

50. Utilisation suivant la revendication 48 ou la revendication 49, dans laquelle la composition comprend en outre un ligand auquel se lie le récepteur.

51. Utilisation suivant la revendication 50, dans laquelle le ligand est un ligand couplé à une toxine, un ligand couplé à une protéine thérapeutique, un ligand marqué avec un radionucléide, un ligand marqué avec un métal ou un ligand qui se lie à un métal.

52. Utilisation suivant l'une quelconque des revendications 29 à 33, dans laquelle la composition comprend en outre :
(a) un composé antimicrobien fusionné à une protéine pour la thérapie d'une tumeur et/ou l'élimination de tumeurs métastatiques ; et/ou
(b) un composé antimicrobien fusionné à une protéine détectable ou une protéine qui induit un signal détectable.

53. Utilisation suivant l'une quelconque des revendications 29 à 33, dans laquelle la composition comprend en outre un composé antimicrobien radiomarqué, un composé antimicrobien marqué avec le métal ou un composé antimicrobien marqué avec une sonde fluorescente.

54. Utilisation suivant la revendication 51 ou la revendication 52, dans laquelle le composé antimicrobien comprend de la lactoferrine.

55. Utilisation suivant la revendication 51, dans laquelle le composé antimicrobien est fusionné à une protéine se liant à un métal.

56. Utilisation suivant l'une quelconque des revendications 29 à 55, dans laquelle la tumeur, le tissu tumoral, le cancer ou la métastase est une tumeur, un tissu tumoral, un cancer ou une métastase du sein, de la prostate, de la vessie, du cerveau, du côlon, du poumon, des ovaires, du pancréas, du foie ou de la peau.
